(19) Europäisches Patentamt
European Patent Office
Office européen des brevets

(11) Veröffentlichungsnummer: **0 186 087**
**B1**

# (12) EUROPÄISCHE PATENTSCHRIFT

(45) Veröffentlichungstag der Patentschrift: **23.08.89**

(51) Int. Cl.⁴: **C 07 D 277/82,** A 61 K 31/425

(21) Anmeldenummer: **85116016.8**

(22) Anmeldetag: **16.12.85**

(54) **Tetrahydro-benzthiazole, deren Herstellung und deren Verwendung als Zwischenprodukte oder als Arznzeimittel.**

(30) Priorität: **22.12.84 DE 3447075**
**13.03.85 DE 3508947**

(43) Veröffentlichungstag der Anmeldung: **02.07.86 Patentblatt 86/27**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen:
**EP-A-0 044 443**
**DE-A-2 136 233**
**US-A-4 208 420**
**US-A-4 337 343**

(73) Patentinhaber: **Dr. Karl Thomae GmbH, Postfach 1755, D-7950 Biberach (Riss) (DE)**

(72) Erfinder: **Griss, Gerhart, Dr. Dipl.- Chem., verstorben (DE)**
Erfinder: **Schneider, Claus, Dr. Dipl.- Chem., Albrecht- Dürer- Strasse 19, D-6507 Ingelheim/Rhein (DE)**
Erfinder: **Hurnaus, Rudolf, Dr. Dipl.- Chem., Silcherstrasse 19, D-7950 Biberach 1 (DE)**
Erfinder: **Kobinger, Walter, Prof. Dr., Belghofergasse 27, A-1121 Wien (AT)**
Erfinder: **Pichler, Ludwig, Dr., Gusshausstrasse 24/11, A-1040 Wien (AT)**
Erfinder: **Bauer, Rudolf, Dr., Aarstrasse 4, D-6200 Wiesbaden (DE)**
Erfinder: **Mierau, Joachim, Dr., An den Weiden 3, D-6500 Mainz 33 (DE)**
Erfinder: **Hinzen, Dieter, Prof. Dr., Konrad-Adenauer- Strasse 26, D-6501 Zornheim (DE)**
Erfinder: **Schingnitz, Günter, Dr., Unter den Gärten, D-6550 Bad Kreuznach 14 (DE)**

**EP 0 186 087 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Aus der Literatur sind zahlreiche Tetrahydro-benzthiazole bekannt. So werden in der US-A-4 337 343 4,5,6,7-Tetrahydro-benzthiazole, die in 2-Stellung durch ein Wasserstoffatom oder eine Alkylgruppe und in 4-, 5- oder 6-Stellung durch eine Alkylaminoalkylgruppe substituiert sind und eine Wirkung auf den Kreislauf aufweisen, in der US-A-4 208 420 4,5,6,7-Tetrahydro-benzthiazole, die in 2-Stellung durch ein Wasserstoffatom oder eine Alkylgruppe und in 4-, 5- oder 6-Stellung durch eine Alkylaminogruppe substituiert sind und u. a. eine stimulierende Wirkung auf das sympathische Nervensystem aufweisen sowie Regulatoren des cerebralen vaskularen Systems darstellen, und in der DE-A-2 136 233 4,5,6,7-Tetrahydro-benzthiazole, die in 2-Stellung durch eine Aminogruppe oder durch einen gegebenenfalls substituierten Ureido- oder Thioureidorest substituiert sind und virustatische Eigenschaften aufweisen, beschrieben.

Es wurde nun gefunden, daß die neuen Tetrahydro-benzthiazole der allgemeinen Formel

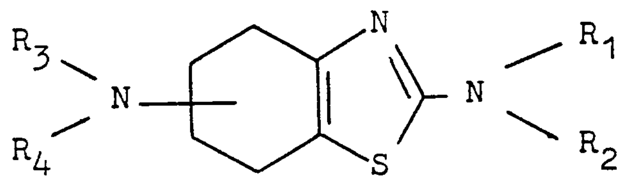 ,(I)

in der

$R_1$ ein Wasserstoffatom, eine Alkylgruppe mit 1 bis 6 Kohlenstoffatomen, eine Alkenyl- oder Alkinylgruppe mit jeweils 3 bis 6 Kohlenstoffatomen, eine Alkanoylgruppe mit 1 bis 6 Kohlenstoffatomen, eine Phenylalkyl- oder Phenylalkanoylgruppe mit jeweils 1 bis 3 Kohlenstoffatomen im Alkylteil, wobei die oben erwähnten Phenylkerne jeweils durch 1 oder 2 Halogenatome substituiert sein können,

$R_2$ ein Wasserstoffatom oder eine Alkylgruppe mit 1 bis 4 Kohlenstoffatomen,

$R_3$ ein Wasserstoffatom, eine Alkylgruppe mit 1 bis 7 Kohlenstoffatomen, eine Cycloalkylgruppe mit 3 bis 7 Kohlenstoffatomen, eine Alkenyl- oder Alkinylgruppe mit jeweils 3 bis 6 Kohlenstoffatomen, eine Alkanoylgruppe mit 1 bis 7 Kohlenstoffatomen, eine Phenylalkyl- oder Phenylalkanoylgruppe mit jeweils 1 bis 3 Kohlenstoffatomen im Alkylteil, wobei der Phenylkern durch Fluor-, Chlor- oder Bromatome substituiert sein kann,

$R_4$ ein Wasserstoffatom, eine Alkylgruppe mit 1 bis 4 Kohlenstoffatomen, eine Alkenyl- oder Alkinylgruppe mit jeweils 3 bis 6 Kohlenstoffatomen oder

$R_3$ und $R_4$ zusammen mit dem dazwischen liegenden Stickstoffatom eine Pyrrolidino-, Piperidino-, Hexamethylenimino- oder Morpholinogruppe bedeuten, wertvolle Eigenschaften aufweisen.

Bedeutet in der obigen allgemeinen Formel I einer der Reste $R_1$ oder $R_3$ oder beide der Reste $R_1$ und $R_3$ einen Acylrest, so stellen diese Verbindungen der allgemeinen Formel I wertvolle Zwischenprodukte zur Herstellung der übrigen Verbindungen der allgemeinen Formel I dar, welche wertvolle pharmakologische Eigenschaften aufweisen, insbesondere eine Wirkung auf das Zentralnervensystem und/oder den Kreislauf.

Gegenstand der vorliegenden Erfindung sind daher neue Tetrahydro-benzthiazole der obigen allgemeinen Formel I deren Enantiomere und deren Säureadditionssalze, insbesondere deren physiologisch verträgliche Säureadditionssalze mit anorganischen oder organischen Säuren, und Verfahren zu ihrer Herstellung.

Bevorzugte Verbindungen der obigen allgemeinen Formel I sind diejenigen, in denen die Gruppe

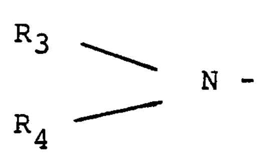

in 5- oder 6-Stellung steht.

Für die bei der Definition der Gruppen

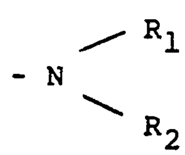

und

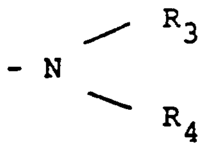

kommt beispielsweise für die

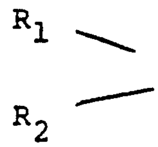

N-Gruppe die Bedeutung Amino-, Methylamino-, Ethylamino-, n-Propylamino-, Isopropylamino-, n-Butylamino-, Isobutylamino-, tert.-Butylamino-, n-Pentylamino-, Isoamylamino-, n-Hexylamino-, Dimethylamino-, Diethylamino-, Di-n-propylamino-, Di-n-butylamino-, Methyl-ethylamino-, Methyl-n-propylamino-, Methyl-isopropylamino-, Ethyl-isopropylamino-, Allylamino-, Buten-2-ylamino-, Hexen-2-ylamino-, N-Methyl-allylamino-, N-Ethyl-allylamino-, N-n-Propyl-allylamino-, N-n-Butyl-allylamino-, Propargylamino-, N-Methylpropargylamino-, N-n-Propyl-propargylamino-, Formylamino-, Acetylamino-, Propionylamino-, -Butanoylamino-, Hexanoylamino-, N-Methyl-acetylamino-, N-Allyl-acetylamino-, N-Propargyl-acetylamino-, Benzylamino-, N-Methyl-benzylamino-, 2-Chlor-benzylamino-, 4-Chlor-benzylamino-, 4-Fluor-benzylamino-, 3,4-Dichlor-benzylamino-, 1-Phenylethylamino-, 2-Phenylethylamino-, 3-Phenyl-n-propylamino-, Benzoylamino-, Phenacetylamino- oder 2-Phenylpropionylaminogruppe und für die

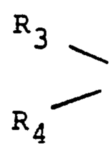

N-Gruppe die der Amino-, Methylamino-, Ethylamino-, n-Propylamino-, Isopropylamino-, n-Butylamino-, Isobutylamino-, tert.-Butylamino-, n-Pentylamino-, Isoamylamino-, n-Hexylamino-, n-Heptylamino-, Dimethylamino-, Diethylamino-, Di-n-propylamino-, Di-n-butylamino-, Methyl-ethylamino-, Methyl-n-propylamino-, Methyl-isopropylamino-, Ethyl-isopropylamino-, Allylamino-, Buten-2-ylamino-, Hexen-2-ylamino-, Diallylamino-, N-Methyl-allylamino-, N-Ethyl-allylamino-, N-n-Propyl-allylamino-, N-n-Butyl-allylamino-, Propargylamino-, Butin-2-ylamino-, Hexin-2-ylamino-, Dipropargylamino-, N-Methyl-propargylamino-, N-Ethyl-propargylamino-, Cyclopropylamino-, Cyclobutylamino-, Cyclopentylamino-, Cyclohexylamino-, Cycloheptylamino-, N-Methylcyclohexylamino-, N-Ethyl-cyclohexylamino-, Formylamino-, Acetylamino-, Propionylamino-, Butanoylamino-, Pentanoylamino-, Hexanoylamino-, Heptanoylamino-, N-Methylacetylamino-, N-Ethyl-acetylamino-, N-n-Propylacetylamino-, N-Allyl-acetylamino-, Benzoylamino-, Fluorbenzoylamino-, Chlorbenzoylamino-, Brombenzoylamino-, Phenylacetylamino-, 2-Phenylpropionylamino-, N-Methyl-benzoylamino-, N-Ethyl-chlorbenzoylamino-, Dichlorbenzoylamino-, N-Cyclohexyl-acetylamino-, Benzylamino-, Chlorbenzylamino-, Brombenzylamino-, 1-Phenylethylamino-, 2-Phenylethylamino-, 2-Phenyl-n-propylamino-, 3-Phenyl-n-propylamino-, N-Methyl-benzylamino-, N-Ethyl-benzylamino-, N-Ethyl-chlorbenzylamino-, N-Ethyl-2-phenylethylamino-, N-Acetyl-benzylamino-, N-Acetyl-chlorbenzylamino-, N-Allyl-benzylamino-, N-Allyl-chlorbenzylamino-, Pyrrolidino-, Piperidino-, Hexamethylenimino- oder Morpholinogruppe in Betracht.

Besonders bevorzugte Verbindungen der obigen allgemeinen Formel I sind jedoch diejenigen Verbindungen der allgemeinen Formel

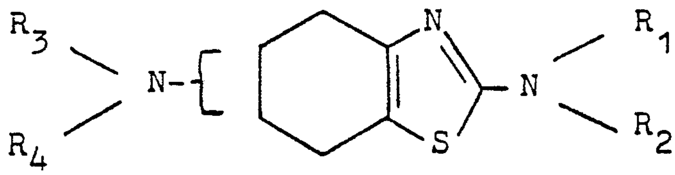

,(Ia)

in der

$R_1$ ein Wasserstoffatom, eine Alkylgruppe mit 1 bis 3 Kohlenstoffatomen, eine Allyl-, Benzyl-, 2-Chlor-benzyl-, 4-Chlor-benzyl-, 3,4-Dichlor-benzyl- oder Phenylethylgruppe,

$R_2$ ein Wasserstoffatom, eine Methyl- oder Ethylgruppe,

$R_3$ ein Wasserstoffatom, eine Alkylgruppe mit 1 bis 6 Kohlenstoffatomen, eine Allyl-, Propargyl-, Benzyl-, Chlorbenzyl-, Phenylethyl-, Cyclopentyl- oder Cyclohexylgruppe,

$R_4$ ein Wasserstoffatom, eine Alkylgruppe mit 1 bis 3 Kohlenstoffatomen oder eine Allylgruppe oder

$R_3$ und $R_4$ zusammen mit dem dazwischen liegenden Stickstoffatom eine Pyrrolidino-, Piperidino-, Hexamethylenimino oder Morpholinogruppe bedeuten, insbesondere jedoch diejenigen Verbindungen, in denen die

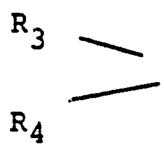

N-Gruppe in 6-Stellung steht, und deren Säureadditionssalze, insbesondere deren physiologisch verträgliche Säureadditionssalze.

Erfindungsgemäß erhält man die neuen Verbindungen nach folgenden Verfahren:

a) Umsetzung eines Cyclohexanons der allgemeinen Formel

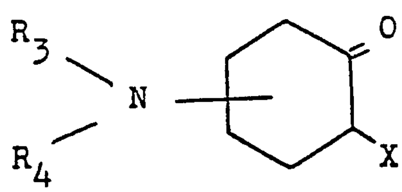

,(II)

in der

$R_3$ und $R_4$ wie eingangs definiert sind und

X eine nukleophile Austrittsgruppe wie ein Halogenatom, z. B. ein Chlor- oder Bromatom, darstellen, mit einem Thioharnstoff der allgemeinen Formel

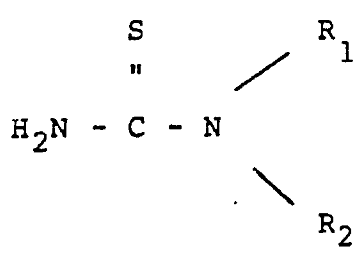

,(III)

in der

$R_1$ und $R_2$ wie eingangs definiert sind.

Die Umsetzung wird in der Schmelze oder in einem Lösungsmittel oder Lösungsmittelgemisch wie Wasser, Ethanol, Wasser/-Ethanol, Pyridin, Dioxan, Dioxan/Wasser, Eisessig, Tetrahydrofuran oder Dimethylformamid zweckmäßigerweise bei Temperaturen zwischen 0 und 150°C, vorzugsweise bei Temperaturen zwischen 20 und 100°C und gegebenenfalls in Gegenwart einer Base, z. B. Natronlauge, Natriumacetat, Pyridin, Triethylamin

oder N-Ethyl-diisopropyldmin durchgeführt. - Die als Ausgangsstoffe verwendeten Verbindungen der allgemeinen Formel II müssen hierbei nicht isoliert werden.

b) Umsetzung einer Verbindung der allgemeinen Formel

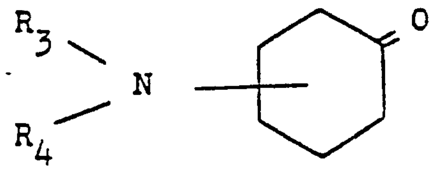

,(IV)

in der
$R_3$ und $R_4$ wie eingangs definiert sind, mit einem Formamidindisulfid der allgemeinen Formel

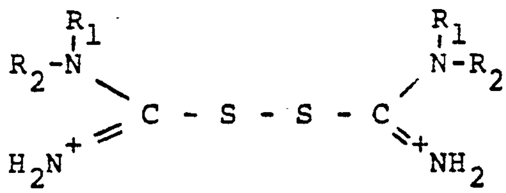

2 $Y^-$ ,(V)

in der
$R_1$ und $R_2$ wie eingangs definiert sind und
$Y^-$ ein Anion einer anorganischen oder organischen Säure darstellt.
Die Umsetzung wird vorzugsweise in der Schmelze oder in einem hochsiedenden Lösungsmittel wie Glykol, Dimethylformamid, Diphenylether oder Dichlorbenzol zweckmäßigerweise bei Temperaturen zwischen 25 und 200°C, vorzugsweise bei Temperaturen zwischen 70 und 150°C, durchgeführt.

c) Zur Herstellung von Verbindungen der allgemeinen Formel I, in der mindestens einer der Reste $R_1$, $R_2$, $R_3$ oder $R_4$ ein Wasserstoffatom darstellt:
Abspaltung eines Schutzrestes von einer Verbindung der allgemeinen Formel

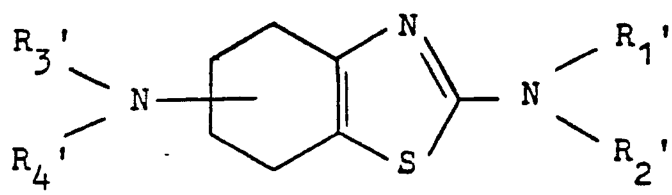

,(VI)

in der
mindestens einer der Reste $R_1'$ $R_2'$, $R_3'$ oder $R_4'$ eine Schutzgruppe für eine Aminogruppe wie eine Acyl- oder Alkoxycarbonylgruppe, z. B. eine Acetyl-, Propionyl-, Methoxycarbonyl- oder Ethoxycarbonylgruppe, oder auch $R_1'$ und $R_2'$ oder $R_3'$ und $R_4'$ zusammen mit dem dazwischen liegenden Stickstoffatom einen Imidorest, z. B. den Phthalimidorest, bedeuten und die übrigen der Reste $R_1'$, $R_2'$, $R_3'$ oder $R_4'$ die für $R_1$ bis $R_4$ eingangs erwähnten Bedeutungen mit Ausnahme der eingangs erwähnten Acylreste bedeuten.
Die Abspaltung eines Schutzrestes erfolgt vorzugsweise hydrolytisch in Gegenwart einer Base wie Natronlauge oder Kalilauge oder in Gegenwart einer Säure wie Salzsäure oder Schwefelsäure in einem wässrigen Lösungsmittel wie Wasser/Ethanol, Wasser/Dioxan oder Wasser/Tetrahydrofuran bei Temperaturen zwischen 50 und 150°C, vorzugsweise bei der Siedetemperatur des Reaktionsgemisches. Ein als Schutzrest verwendeter Imidorest wie der Phthalimidorest wird vorzugsweise mit Hydrazin in einem Lösungsmittel wie Wasser, Wasser/Ethanol oder Wasser/Dioxan bei der Siedetemperatur des verwendeten Lösungsmittels abgespalten.

d) Zur Herstellung von Verbindungen der allgemeinen Formel I, in der mindestens einer der Reste $R_1$, $R_2$, $R_3$ oder $R_4$ eine der eingangs erwähnten Alkyl- oder Phenylalkylgruppen bedeutet:
Reduktion einer Verbindung der allgemeinen Formel

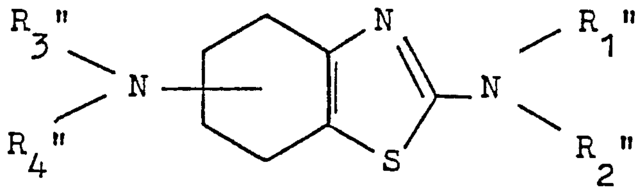

,(VII)

in der

mindestens einer der Reste $R_1''$, $R_2''$, $R_3''$ oder $R_4''$ einen der eingangs erwähnten Acyl- oder Phenylacylreste darstellt und die übrigen der Reste die für $R_1$, $R_2$, $R_3$ und $R_4$ eingangs erwähnten Bedeutungen besitzen, mit einem Metallhydrid in einem Lösungsmittel.

Die Reduktion wird in einem geeigneten Lösungsmittel wie Diethylether, Tetrahydrofuran, Glykoldimethylether oder Dioxan mit einem Metallhydrid, z. B. mit einem komplexen Metallhydrid wie Lithiumaluminiumhydrid, bei Temperaturen zwischen 0 und 100°C, vorzugsweise jedoch bei Temperaturen zwischen 20 und 80°C, durchgeführt.

Zur Herstellung von Verbindungen der allgemeinen Formel I, in der $R_3$ eine der eingangs erwähnten Acylgruppen darstellt, wird die Umsetzung besonders vorteilhaft mit Lithiumaluminiumhydrid bei Temperaturen zwischen 0 und 30°C, vorzugsweise bei Raumtemperatur, durchgeführt.

e) Zur Herstellung von Verbindungen der allgemeinen Formel I, in der mindestens einer der Reste $R_1$, $R_2$, $R_3$ oder $R_4$ eine der eingangs erwähnten Alkyl-, Cycloalkyl-, Alkenyl-, Alkinyl- oder Phenylalkylgruppen bedeutet:

Umsetzung einer Verbindung der allgemeinen Formel

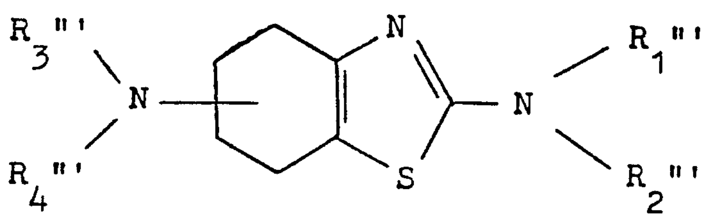

,(VIII)

in der

mindestens einer der Reste $R_1'''$, $R_2'''$, $R_3'''$ oder $R_4'''$ ein Wasserstoffatom darstellt und die übrigen der Resten $R_1'''$, $R_2'''$, $R_3'''$ oder $R_4'''$ die für $R_1$ bis $R_4$ eingangs erwähnten Bedeutungen besitzen, mit einer Verbindung der allgemeinen Formel

$$R_5 - Z \qquad ,(IX)$$

in der $R_5$ eine der für $R_1$ bis $R_4$ eingangs erwähnten Alkyl-, Cycloalkyl-, Alkenyl-, Alkinyl- oder Phenylalkylgruppen und

Z eine nukleophile Austrittsgruppe wie ein Halogenatom oder einen Sulfonsäurerest, z. B. ein Chlor-, Brom- oder Jodatom, eine Methoxysulfonyloxy- oder p-Toluolsulfonyloxygruppe,

oder Z zusammen mit einem benachbarten Wasserstoffatom des Restes $R_5$ ein Sauerstoffatom bedeuten.

Die Umsetzung wird in einem Lösungsmittel wie Wasser, Methanol, Ethanol, Tetrahydrofuran, Dioxan, Aceton, Acetonitril oder Dimethylsulfoxid mit einem Alkylierungsmittel wie Methyljodid, Dimethylsulfat, Ethylbromid, Diethylsulfat, Allyljodid, Benzylbromid, 2-Phenylethylbromid oder Methyl-p-toluolsulfonat, gegebenenfalls in Gegenwart einer Base wie Natronlauge, Kaliumcarbonat, Natriumhydrid, Kalium-tert.-butylat oder Triethylamin zweckmäßigerweise bei Temperaturen zwischen -10 und 50°C, vorzugsweise jedoch bei Temperaturen zwischen 0 und 30°C, durchgeführt. Die Umsetzung kann jedoch auch ohne Lösungsmittel durchgeführt werden.

Die Alkylierung des Stickstoffatoms kann auch mittels Formaldehyd/Ameisensäure bei erhöhten Temperaturen, z. B. bei der Siedetemperatur des Reaktionsgemisches, oder mit einer entsprechenden Carbonylverbindung und einem komplexen Metallhydrid wie Natriumborhydrid oder Natriumcyanborhydrid in einem Lösungsmittel wie Wasser/Methanol, Ethanol, Ethanol/Wasser, Dimethylformamid oder Tetrahydrofuran bei Temperaturen zwischen 0 und 50°C, vorzugsweise jedoch bei Raumtemperatur, durchgeführt werden.

Erhält man erfindungsgemäß eine Verbindung der allgemeinen Formel I, in der mindestens einer der Reste $R_1$ oder $R_3$ ein Wasserstoffatom darstellen, so kann diese mittels entsprechende Acylierung in eine entsprechende Verbindung der allgemeinen Formel I, in der mindestens einer der Reste $R_1$ oder $R_3$ einen der eingangs erwähnten Acylreste darstellt, übergeführt werden.

Die nachträgliche Acylierung wird zweckmäßigerweise in einem Lösungsmittel wie Methylenchlorid, Chloroform, Tetrachlorkohlenstoff, Ether, Tetrahydrofuran, Dioxan, Eisessig, Benzol, Toluol, Acetonitril oder Dimethylformamid, gegebenenfalls in Gegenwart eines die Säure aktivierenden Mittels oder eines wasserentziehenden Mittels, z. B. in Gegenwart von Chlorameisensäureethylester, Thionylchlorid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z. B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen -25°C und 250°C, vorzugsweise jedoch bei Temperaturen zwischen -10°C und der Siedetemperatur des verwendeten Lösungsmittels, durchgeführt. Die Umsetzung kann auch ohne Lösungsmittel durchgeführt werden, desweiteren kann während der Umsetzung entstehendes Wasser durch azeotrope Destillation, z. B. durch Erhitzen mit Toluol am Wasserabscheider, oder durch Zugabe eines Trockenmittels wie Magnesiumsulfat oder Molekularsieb abgetrennt werden.

Die erhaltenen Verbindungen der allgemeinen Formel I, welche mindestens ein chirales Zentrum enthalten, lassen sich in ihre Enantiomeren nach üblichen Methoden auftrennen, beispielsweise durch Säulenchromatographie an einer chiralen Phase, mittels fraktionierter Kristallisation ihrer diastereomeren Salze oder mittels Säulenchromatographie ihrer Konjugate mit optisch aktiven Hilfssäuren wie Weinsäure, 0,0-Dibenzoylweinsäure, Camphersäure, Camphersulfonsäure oder α-Methoxy-phenylessigsäure.

Ferner lassen sich die erhaltenen Verbindungen in ihre Säureadditionssalze, insbesondere in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure in Betracht.

Die als Ausgangsstoffe verwendeten Verbindungen der allgemeinen Formeln II bis IX sind teilweise literaturbekannt bzw. man erhält diese nach literaturbekannten Verfahren.

So erhält man beispielsweise eine Verbindung der allgemeinen Formel II durch Halogenierung des entsprechenden Cyclohexanons, welches seinerseits durch Oxidation des entsprechenden Cyclohexanols und gegebenenfalls anschließende Alkylierung und/oder Acylierung hergestellt wird.

Die als Ausgangsstoffe verwendeten Verbindungen der allgemeinen Formeln VI, VII und VIII erhält man durch Kondensation eines entsprechenden α-Brom-cyclohexanons mit einem entsprechenden Thioharnstoff.

Wie bereits eingangs erwähnt, stellen die Verbindungen der allgemeinen Formel I, in der mindestens einer der Reste $R_1$ bis $R_4$ einen der eingangs erwähnten Acylreste darstellt, wertvolle Zwischenprodukte zur Herstellung der Verbindungen der allgemeinen Formel I dar, in denen $R_1$ bis $R_4$ mit Ausnahme der eingangs erwähnten Acylreste die für $R_1$ bis $R_4$ eingangs erwähnten Bedeutungen aufweisen. Diese Verbindungen und deren physiologisch verträgliche Säureadditionssalze weisen wertvolle pharmakologische Eigenschaften auf, insbesondere eine Wirkung auf den Blutdruck, eine herzfrequenzsenkende Wirkung und eine Wirkung auf das Zentralnervensystem, insbesondere eine Dopaminrezeptor-stimulierende Wirkung.

Beispielsweise wurde daher bei den Verbindungen

A = 2-Amino-6-dimethylamino-4,5,6,7-tetrahydro-benzthiazol-dihydrochlorid,
B = 2-Amino-6-pyrrolidino-4,5,6,7-tetrahydro-benzthiazol-dihydrochlorid,
C = 2-Amino-6-n-propylamino-4,5,6,7-tetrahydro-benzthiazol-dihydrochlorid,
D = 2-Allylamino-6-dimethylamino-4,5,6,7-tetrahydro-benzthiazol-dihydrochlorid,
E = 6-[N-Allyl-N-(4-chlor-benzyl)-amino]-2-amino-4,5,6,7-tetrahydro-benzthiazol-dihydrochlorid und
F = 2-Amino-6-diallylamino-4,5,6,7-tetrahydro-benzthiazol-dihydrochlorid

zur Untersuchung der Beeinflussung praesynaptischer Dopaminrezeptoren zunächst die Wirkung auf die exploratorische Aktivität von Mäusen untersucht und anschließend, nach Abklärung einer Wirkung auf postsynaptische Dopaminrezeptoren (Motilität an mit Reserpin vorbehandelten Tieren), die Beeinflussung des Dopaminturnovers und der Dopaminsynthese wie folgt bestimmt:

**1. Hemmung der exploratorischen Aktivität**

Die Aktivitätsmessung erfolgte in Beobachtungskäfigen, die mit einer Infrarotlichtschranke versehen sind. Gemessen wird die Häufigkeit der Unterbrechung des Lichtstrahles durch eine Gruppe von 5 Mäusen innerhalb von 5 Minuten. Gruppen von jeweils 5 Tieren erhalten die zu untersuchende Substanz, falls nichts anderes angegeben ist, in einer Dosis von 10 mg/kg subkutan injiziert. 1 Stunde später werden die Tiere in die Beobachtungskäfige gebracht, wo sofort mit der Messung der exploratorischen Aktivität über 5 Minuten begonnen wird. Parallel bzw. alternierend zu Gruppen, die mit Testsubstanz behandelt wurden, werden kochsalzbehandelte Kontrollgruppen (0,9-%-ig; 0,1 ml/10 g Körpergewicht subkutan) untersucht.

Die Ergebnise sind in folgender Tabelle zusammengestellt:

| Substanz | Dosis (mg/kg s.c.) | Hemmung der Aktivität in Prozent gegenüber kochsalzbehandelten Kontrollen |
|---|---|---|
| A | 2,71[1] | 50 |
| B | 10,0 | 94 |
| C | 10,0 | 20[2] |
| D | 10,0 | 76[2] |
| E | 10,0 | 56[2] |
| F | 10,0 | 60[2] |

1) abgelesen von Dosis Wirkungskurve im Bereich 1 - 10 mg/kg subkutan.
2) Explorationsmessung: 75 Minuten nach Substanzapplikation.

**2. Bestimmung der Dopaminturnoverhemmung**

Die Dopaminturnoverhemmung wurde an Mäusen gemessen. Bei Tieren, die mit α-Methylparatyrosin (AMPT) (250 mg/kg intraperitoneal) zum Zeitpunkt 15 Minuten des Experimentes behandelt werden, fällt die Dopaminkonzentration im Gesamthirn mit fortschreitender Versuchsdauer ab. Durch Substanzen, die an Autorezeptoren wirken, kann der Dopaminabfall (im Vergleich zu mit Kochsalzlösung behandelten Kontrolltieren) verhindert werden.

Testsubstanzen werden zum Zeitpunkt 0 des Experiments - soweit nicht anders angegeben - mit 5 mg/kg s.c. appliziert. Zum Zeitpunkt 4 Stunden und 15 Minuten des Experiments werden die Tiere getötet und die Gehirne der Dopaminbestimmung mittels Hochdruckflüssigkeitchromatographie mit elektrochemischer Detektion zugeführt. Bestimmt wird die durch die Testsubstanz bewirkte prozentuelle Hemmung des durch AMPT induzierten Dopaminabfalls.

| Substanz | Dosis (mg/kg s.c.) | % Hemmung des AMPT-Effektes |
|---|---|---|
| A | 0,951[1] | 50 |
| B | 5 | 67 |
| D | 5 | 52 |
| E | 5 | 32 |

1) abgelesen von Dosis Wirkungskurve im Bereich von 0,5 - 3 mg/kg s.c.

**3. Bestimmung der Dopaminsynthesehemmung**

Hierzu erhalten 5 Tiere, sofern nicht anders angegeben ist, jeweils 10 mg/kg s.c. der zu untersuchenden Substanz. Nach 5 Minuten erfolgt die Gabe von 750 mg/kg i.p. γ-Butyrolacton, um über Blockade der präsynaptischen Impulsleitung den Einfluß postsynaptischer Rückkopplungsschleifen auf die Dopaminsyntheserate auszuschließen. Dies führt zu einer beträchtlichen Steigerung der DOPA- bzw. Dopaminsynthese. Zur Hemmung der Decarboxylierung von DOPA werden nach weiteren 5 Minuten 200 mg/kg i.p. 3-Hydroxybenzylhydrazin-hydrochlorid appliziert. 40 Minuten nach Substanzgabe werden die Tiere getötet und das Corpus striatum präpariert. Die Messung des DOPA-Gehaltes erfolgt mit Hilfe von HPLC mit elektrochemischer Detektion (Standard: Dihydroxibenzylamin).

Bestimmt wird die durch die Testsubstanz bewirkte prozentuelle Hemmung der durch γ-Butyrolacton stimulierten Dopaakkumulierung gegenüber den mit 0,9-%-iger Kochsalzlösung behandelten Kontrolltieren.

Die Ergebnisse dieses Versuches sind in nachfolgender Tabelle zusammengestellt:

| Substanz | Dosis (mg/kg s.c.) | Hemmung der Dopaakkumulierung in % gegenüber kochsaltzbehandelten Kontrollen |
|---|---|---|
| A | 0,55[1] | 50 |
| C | 10 | 60 |

1) abgelesen von Dosiswirkungskurve im Bereich 0,1 - 1,0 mg/kg subkutan.

**4. Bestimmung der Anti-Parkinsonismus- bzw. der Anti-Parkinson-Wirkung**

Die Entdeckung des Neurotoxins 1-Methyl-4-phenyl-1,2,3,6-tetrahydro-pyridin (MPTP) (Langston et al., Science 219, (1983) 979) hat ein Tiermodell für die Parkinson'sche-Erkrankung zur Verfügung gestellt.

Das durch MPTP beim Menschen und beim Affen ausgelöste, irreversible, neurologische Krankheitsbild ähnelt in seiner klinischen, pathologischen, biochemischen und pharmakologischen Ausprägung weitgehend der idiopathischen Parkinson'schen Erkrankung (Markey et al., Nature 311, (1984) 464). Ursache dieser überzeugenden Übereinstimmung ist die Tatsache, daß MPTP selektiv jene kleine Gruppe dopaminerger Nervenzellen in der Substantia nigra des Gehirns zerstört, welche auch bei der natürlich auftretenden Parkinson'schen Erkrankung durch degenerative Prozesse zerstört werden. Es wird gar diskutiert, daß auch die Ursache der idiopathischen Parkinson'schen Erkrankung im Organismus entstehendes MPTP oder eine ähnliche chemische Verbindung ist (Snyder, S.H., Nature 311, (1984) 514). Möglicherweise bedingt durch den spezifischen Metabolismus des MPTP ist die klinische Ausprägung des MPTP-Parkinson'bildes bisher außer beim Menschen nur beim Affen nachweisbar.

Das an Rhesusaffen verwirklichte MPTP-Modell ist daher in hervorragendem Maße geeignet, die Wirkung von Anti-Parkinson-Medikamenten zu prüfen. 7 Rhesusaffen wurden mit MPTP (3 Tage lang 1 x 0,15 mg/kg i.m. täglich, 3 Tage Pause, 3 Tage lang 1 x 0,30 - 0,40 mg/kg täglich) appliziert und wiesen folgende Symptome auf: die Tiere waren akinetisch und nicht in der Lage, Wasser und Futter aufzunehmen. Sie zeigten eine typische gebeugte Haltung; gelegentlich traten kataleptische Zustände auf. Die Extremitäten wiesen einen Rigor auf, welcher bei passiver Bewegung von klonischen Krämpfen durchbrochen wurde. Willkürbewegungen des Rumpfes und der Extremitäten waren in der Regel durch stärkste, schmerzhafte Reize nicht auszulösen.

Nach der intramuskulären Gabe von Verbindung C (10 - 100 µg) traten im zeitlichen Abstand von 5 bis 10 min erste Willkürbewegungen auf, die in den folgenden 10 bis 30 min von einer allmählichen, weitestgehenden Normalisierung der Motorik gefolgt war. Die Tiere waren in der Lage Nahrung aufzunehmen. Sie verhielten sich innerhalb ihrer Käfige regelrecht, dies galt auch hinsichtlich Vigilanz und artspezifischen Verhaltens. Als Restsymptomik wurden gelegentlich vorübergehender, leichter Ruhetremor und Verringerung der groben Kraft registriert. Eine Sedation trat nicht ein. Die Hautdurchblutung erschien gegenüber dem Zustand vor Gabe der Verbindung C gesteigert.

Die Wirkung von Verbindung C ließ nach ca. 5 bis 7 Stunden nach, und die Tiere verfielen wieder in die oben beschriebene Parkinson-Symptomatik; eine erneute Applikation dieser Verbindung führt wieder zur Besserung bzw. weitgehenden Aufhebung der klinisch pathologischen Erscheinung. Die vorteilhafte Wirkung der Verbindung wurde an jedem einzelnen Tier somit mehrfach reproduziert.

Nebenwirkungen traten in den bisher angewandten Dosierungen nicht in Erscheinung.

Ferner sind die erfindungsgemäß hergestellten Verbindungen weitgehend untoxisch. So konnten bei der Untersuchung der Substanzen an Mäusen mit Dosen zwischen 27 und 50 mg/kg s.c. keine Todesfälle festgestellt werden.

Aufgrund ihrer pharmakologischen Eigenschaften eignen sich die erfindungsgemäß hergestellten Verbindungen der allgemeinen Formel I und deren physiologisch verträgliche Säureadditionssalze zur Behandlung von zentralnervösen, neuropsychiatrischen Erkrankungen, insbesondere der Schizophrenie, zur Behandlung des Parkinsonismus bzw. Parkinson'schen-Erkrankung und/oder zur Behandlung von Kreislauferkrankungen, insbesondere der Hypertonie.

Zur pharmazeutischen Anwendung lassen sich die neuen Verbindungen und deren physiologisch verträgliche Säureadditionssalze, gegebenenfalls in Kombination mit anderen Wirkstoffen, in die üblichen galenischen Anwendungsformen wie Dragées, Tabletten, Pulver, Suppositorien, Suspensionen, Tropfen oder Ampullen einarbeiten. Die Einzeldosis beträgt hierbei 1 bis 4 x täglich 0,01 - 0,5 mg/kg Körpergewicht, vorzugsweise jedoch 0,1 - 0,3 mg/kg Körpergewicht.

Die nachfolgenden Beispiele sollen die Erfindung näher erläutern:

**Beispiel A**

4-[N-(4-Chlor-benzyl)-amino]-cyclohexanol

75,8 g (0,5 Mol) 4-Amino-cyclohexanol-hydrochlorid werden in 60 ml Wasser gelöst und nach Zugabe von 36 g (0,26 Mol) Kaliumcarbonat und 500 ml Toluol bis zur Beendigung der Wasserabscheidung am Wasserabscheider gekocht. Dann werden unter weiterem Kochen am Wasserabscheider langsam 71,7 g (0,5 Mol) 4-Chlorbenzaldehyd zugegeben. Nachdem sich die berechnete Menge Wasser abgeschieden hat, wird auf Wasser gegeben und die Toluolphase abgetrennt und eingeengt. Der Einengungsrückstand wird in 500 ml Ethanol gelöst und unter Rühren portionsweise mit 19 g (0,5 Mol) Natriumborhydrid versetzt. Nach Stehen über Nacht wird eingeengt, mit Wasser versetzt und mit Chloroform extrahiert. Nach dem Trocknen und Einengen der Extrakte wird der Rückstand aus Essigsäure-ethylester umkristallisiert.

Ausbeute: 93,4 g (78 % der Theorie),
Schmelzpunkt: 103 - 104°C

| | | | | |
|---|---|---|---|---|
| Ber.: | C 65,12 | H 7,57 | N 5,84 | Cl 14,79 |
| Gef.: | 65,21 | 7,68 | 5,93 | 14,65 |

Analog Beispiel A wurde unter Verwendung von Propionaldehyd folgende Verbindung hergestellt:

4-n-Propylamino-cyclohexanol

Ausbeute: 12,4 % der Theorie,
Schmelzpunkt: < 20° C
Ber.: m/e = 157
Gef.: m/e = 157

**Beispiel B**

4-[N-(4-Chlor-benzyl)-methylamino]-cyclohexanol
7,2 g (30 mMol) 4-[N-(4-Chlor-benzyl)-amino]-cyclohexanol werden in 30 ml Dimethylformamid gelöst und nach Zugabe von 2,2 g (16 mMol) Kaliumkarbonat tröpfenweise mit 4,26 g (30 mMol) Methyljodid versetzt. Nach Abklingen der leicht exothermen Reaktion wird eingeengt, mit Wasser versetzt und mit Chloroform extrahiert. Die eingeengten Extrakte werden zur Reinigung an Kieselgel chromatographiert (Fließmittel: Methylenchlorid/Methanol = 20/1).

Ausbeute: 3,3 g (43,4 % der Theorie),
Schmelzpunkt: 74 - 75° C

| | | | | |
|---|---|---|---|---|
| Ber.: | C 66,26 | H 7,94 | N 5,52 | Cl 13,97 |
| Gef.: | 66,36 | 7,95 | 5,46 | 13,81 |

Analog Beispiel B wurden folgende Verbindungen hergestellt:

4-Hexamethylenimino-cyclohexanol
Hergestellt aus 4-Amino-cyclohexanol und 1,6-Dibromhexan.
Ausbeute: 47,3 % der Theorie,
Schmelzpunkt: < 20° C
Ber.: m/e = 197
Gef.: m/e = 197

4-Diallylamino-cyclohexanol
Hergestellt aus 4-Aminocyclohexanol und Allylbromid.
Ausbeute: 51 % der Theorie,
Schmelzpunkt: < 20° C
Ber.: m/e = 195
Gef.: m/e = 195

4-Piperidino-cyclohexanol
Hergestellt aus 4-Amino-cyclohexanol und 1,5-Dibrompentan.
Ausbeute: 65,8 % der Theorie,
Schmelzpunkt: < 20° C
Ber.: m/e = 183
Gef.: m/e = 183

4-Pyrrolidino-cyclohexanol
Hergestellt aus 4-Amino-cyclohexanol und 1,4-Dibrom-butan.
Ausbeute: 35,8 % der Theorie,
Schmelzpunkt: < 20° C
Ber.: m/e = 169
Gef.: m/e = 169

**Beispiel C**

4-Diethylamino-cyclohexanol
28,75 g (0,25 Mol) 4-Amino-cyclohexanol werden unter Zusatz von 20 g (0,5 Mol) Natriumhydroxid in 150 ml Wasser gelöst und tropfenweise mit 65,6 ml (0,5 Mol) Diethylsulfat versetzt. Hierbei erwärmt sich der Ansatz auf 65° C. Man rührt eine Stunde bei 70° C nach, gibt dann auf Eis und extrahiert mit Chloroform.

Ausbeute: 18,2 g (42,5 % der Theorie),
Schmelzpunkt: < 20°C
Ber.: m/e = 171
Gef.: m/e = 171

**Beispiel D**

4-[N-(4-Chlor-benzyl)-amino]-cyclohexanon

23,9 g (0,1 Mol) 4-[N-(4-Chlorbenzyl)-amino]-cyclohexanol werden in 125 ml Eiswasser suspendiert und mit 32 ml konzentrierter Schwefelsäure versetzt. Anschließend gibt man 29,4 g (0,1 Mol) Kaliumdichromat in 2 Portionen zu und erwärmt 5 Stunden auf 50°C. Dann kühlt man ab, stellt mit Natronlauge alkalisch und extrahiert mit Chloroform. Nach dem Einengen erhält man eine gelblich gefärbte ölige Flüssigkeit.
Ausbeute: 8,2 g (34 % der Theorie),
Schmelzpunkt: < 20°C
Ber.: m/e = 237/239
Gef.: m/e = 237/239

Analog Beispiel D wurden folgende Verbindungen hergestellt:

4-[N-(4-Chlor-benzyl)-methylamino]-cyclohexanon
Ausbeute: 38 % der Theorie,
Schmelzpunkt: < 20°C
Ber.: m/e = 251/253
Gef.: m/e = 251/253

4-Diallylamino-cyclohexanon
Ausbeute: 21 % der Theorie,
Schmelzpunkt: < 20°C
Ber.: m/e = 193
Gef.: m/e = 193

4-Piperidino-cyclohexanon
Ausbeute: 22,2 % der Theorie,
Schmelzpunkt: < 20°C
Ber.: m/e = 181
Gef.: m/e = 181

4-Pyrrolidino-cyclohexanon
Ausbeute: 45,1 % der Theorie,
Schmelzpunkt: < 20°C
Ber.: m/e = 167
Gef.: m/e = 167

4-Diethylamino-cyclohexanon
Ausbeute: 49,7 % der Theorie,
Schmelzpunkt: < 20°C
Ber.: m/e = 169
Gef.: m/e = 169

4-n-Propylamino-cyclohexanon
Ausbeute: 33 % der Theorie,
Schmelzpunkt: < 20°C
Ber.: m/e = 155
Gef.: m/e = 155

**Beispiel E**

4-[N-(4-Chlor-benzyl)-methylamino]-cyclohexanon

8,4 g (35 mMol) 4-[N-(4-Chlor-benzyl)-amino]-cyclohexanon werden in 50 ml absolutem Dimethylformamid gelöst und nach Zugabe von 2,6 g (18,7 mMol) Kaliumcarbonat bei 25 - 30°C tropfenweise mit 5,0 g (35 mMol) Methyljodid versetzt. Nach Stehen über Nacht wird eingeengt, mit Wasser versetzt und mit Chloroform extrahiert. Die Extrakte werden getrocknet und eingeengt.

Ausbeute: 8,1 g (93 % der Theorie),
Schmelzpunkt: < 20° C
Ber.: m/e = 251/253
Gef.: m/e = 251/253

Analog Beispiel E wurden folgende Verbindungen hergestellt:

4-[N-Allyl-N-(4-chlor-benzyl)-amino]-cyclohexanon
Ausbeute: 70,7 % der Theorie
Schmelzpunkt: < 20° C
Ber.: m/e = 277/279
Gef.: m/e = 277/279

4-[N-(4-Chlor-benzyl)-ethylamino]-cyclohexanon
Ausbeute: 30 % der Theorie
Schmelzpunkt: < 20° C
Ber.: m/e = 265/267
Gef.: m/e = 265/267

**Beispiel F**

4-Hexamethylenimino-cyclohexanon
Zur Suspension von 107,5 g (0,5 Mol) Pyridiniumchlorochromat und 40 g (0,5 Mol) Natriumacetat in 700 ml Methylenchlorid tropft man bei 20 - 25° C die Lösung von 47 g (0,5 Mol) 4-Hexa-methylenimino-cyclohexanol in 300 ml Methylenchlorid. Nach einstündigem Rühren bei 20° C gibt man auf Eiswasser und Natronlauge und extrahiert mit Methylenchlorid. Nach dem Trocknen und Einengen der Extrakte hinterbleibt eine farblose ölige Flüssigkeit.

Ausbeute: 16,8 g (35,8 % der Theorie),
Schmelzpunkt: < 20° C
Ber.: m/e = 195
Gef.: m/e = 195

**Beispiel 1**

2-Amino-6-dimethylamino-4,5,6,7-tetrahydro-benzthiazol-dihydrochlorid
2,82 g (0,02 Mol) 4-Dimethylamino-cyclohexanon werden in 20 ml Eisessig gelöst, mit 4,7 ml 36-%-iger Bromwasserstoffsäure in Eisessig versetzt und unter Kühlung tropfenweise mit einer Lösung von 1,0 ml (0,02 Mol) Brom in 12 ml Eisessig behandelt. Anschließend engt man im Vakuum ein und digeriert den Rückstand mehrfach mit Diethylether. Die Etherextrakte werden verworfen und der Rückstand in 50 ml Ethanol gelöst. Nach Zugabe von 3,04 g (40 mMol) Thioharnstoff wird 5 Stunden am Rückfluß gekocht. Dann wird eingeengt, mit Natronlauge alkalisch gestellt und mit Chloroform extrahiert. Nach dem Trocknen und Einengen der Extrakte wird an Kieselgel säulenchromatographisch gereinigt (Fließmittel: Chloroform/Methanol = 1/1). Anschließend wird die Base (Schmelzpunkt: 191° C) in Aceton gelöst und mit isopropanolischer Salzsäure in das Dihydrochlorid überführt.

Ausbeute: 1,09 g (20 % der Theorie),
Schmelzpunkt: 272° C

| | | | | |
|---|---|---|---|---|
| Ber.: | C 40,00 | H 6,34 | N 15,55 | Cl 26,24 |
| Gef.: | 39,63 | 6,55 | 15,31 | 26,29 |

Aus den entsprechenden Ketonen wurden analog Beispiel 1 die nachfolgenden Tetrahydrobenzthiazole hergestellt:

2-Amino-6-diethylamino-4,5,6,7-tetrahydro-benzthiazol
Ausbeute: 25 % der Theorie,
Schmelzpunkt: 182 - 183° C

| | | | |
|---|---|---|---|
| Ber.: | C 58,62 | H 8,49 | N 18,64 |
| Gef.: | 58,65 | 8,72 | 18,50 |

2-Amino-6-piperidino-4,5,6,7-tetrahydro-benzthiazol-dihydrochlorid
Ausbeute: 13 % der Theorie,
Schmelzpunkt: 280° C

| | | | | |
|---|---|---|---|---|
| Ber.: | C 46,45 | H 6,82 | N 13,55 | Cl 22,85 |
| Gef.: | 46,37 | 6,75 | 13,41 | 22,95 |

2-Amino-6-pyrrolidino-4,5,6,7-tetrahydro-benzthiazol
Ausbeute: 24,4 % der Theorie,
Schmelzpunkt: 204 - 206° C

| | | | |
|---|---|---|---|
| Ber.: | C 59,15 | H 7,67 | N 18,81 |
| Gef.: | 59,50 | 7,74 | 18,95 |

2-Amino-6-diallylamino-4,5,6,7-tetrahydro-benzthiazol-dihydrochlorid
Ausbeute: 19 % der Theorie,
Schmelzpunkt: 242° C

| | | | | |
|---|---|---|---|---|
| Ber.: | C 48,44 | H 6,56 | N 13,03 | Cl 22,00 |
| Gef.: | 47,90 | 6,49 | 12,95 | 22,21 |

2-Amino-6-[N-(4-chlor-benzyl)-amino]-4,5,6,7-tetrahydro-benzthiazol
Ausbeute: 35 % der Theorie,
Schmelzpunkt: 146° C

| | | | | |
|---|---|---|---|---|
| Ber.: | C 57,23 | H 5,49 | N 14,30 | Cl 12,06 |
| Gef.: | 56,93 | 5,56 | 13,86 | 12,04 |

2-Amino-6-[N-(4-chlor-benzyl)-methylamino]-4,5,6,7-tetrahydro-benzthiazol
Ausbeute: 36 % der Theorie,
Schmelzpunkt: 163° C

| | | | | |
|---|---|---|---|---|
| Ber.: | C 58,69 | H 5,89 | N 13,64 | Cl 11,51 |
| Gef.: | 58,50 | 5,94 | 13,49 | 11,55 |

2-Amino-6-[N-(4-chlor-benzyl)-ethylamino]-4,5,6,7-tetrahydro-benzthiazol-dihydrochlorid
Ausbeute: 49 % der Theorie,
Schmelzpunkt: 258° C (Zersetzung)

| | | | | |
|---|---|---|---|---|
| Ber.: | C 48,67 | H 5,61 | N 10,64 | Cl 26,94 |
| Gef.: | 48,30 | 5,85 | 10,57 | 26,97 |

2-Amino-6-[N-allyl-N-(4-chlor-benzyl)-amino]-4,5,6,7-tetrahydro-benzthiazol-dihydrochlorid
Ausbeute: 46,5 % der Theorie,
Schmelzpunkt: 240° C (Zersetzung)

| | | | | |
|---|---|---|---|---|
| Ber.: | C 50,19 | H 5,45 | N 10,33 | Cl 26,14 |
| Gef.: | 49,84 | 5,68 | 9,97 | 26,04 |

2-Amino-6-hexamethylenimino-4,5,6,7-tetrahydro-benzthiazol-dihydrochlorid
Ausbeute: 15,4 % der Theorie,
Schmelzpunkt: 295° C (Zersetzung)

| | | | | |
|---|---|---|---|---|
| Ber.: | C 48,17 | H 7,14 | N 12,95 | Cl 21,86 |
| Gef.: | 47,90 | 7,34 | 12,44 | 21,64 |

2-Allylamino-6-dimethylamino-4,5,6,7-tetrahydro-benzthiazol-dihydrochlorid
Hergestellt aus 4-Dimethylamino-cyclohexanon durch Bromierung und anschließende Umsetzung mit Allylthioharnstoff.
Ausbeute: 64 % der Theorie,
Schmelzpunkt: 248° C

| | | | | |
|---|---|---|---|---|
| Ber.: | C 46,45 | H 6,82 | N 13,54 | Cl 22,85 |
| Gef.: | 46,30 | 7,00 | 13,29 | 22,99 |

2-Amino-5-dimethylamino-4,5,6,7-tetrahydro-benzthiazol-dihydrochlorid
Hergestellt aus 3-Dimethylamino-cyclohexanon.
Ausbeute: 33 % der Theorie,
Schmelzpunkt: 194° C

| | | | | |
|---|---|---|---|---|
| Ber.: | C 40,00 | H 6,34 | N 15,55 | Cl 26,24 |
| Gef.: | 39,74 | 6,37 | 15,15 | 25,96 |

2-Amino-5-morpholino-4,5,6,7-tetrahydro-benzthiazol-dihydrochlorid
Hergestellt aus 3-Morpholino-cyclohexanon
Ausbeute: 7,4 g (20 % der Theorie),
Schmelzpunkt: 237 - 238° C

| | | | |
|---|---|---|---|
| Ber.: | C 42,31 | H 6,13 | N 13,46 |
| Gef.: | 42,00 | 6,29 | 13,13 |

**Beispiel 2**

2,6-Diamino-4,5,6,7-tetrahydro-benzthiazol-dihydrochlorid

a) 4-(Phthalimido)-cyclohexanol
75 5 g (0,5 Mol) 4-Aminocyclohexanol-hydrochlorid und 74,0 g (0,5 Mol) Phthalsäureanhydrid werden nach Zusatz von 65 g (0,5 Mol) Ethyl-diisopropyl-amin und 1000 ml Toluol 36 Stunden am Wasserabscheider gekocht. Dann wird mit Wasser versetzt, die Toluolphase abgetrennt und die wässrige Phase mehrmals mit Chloroform extrahiert. Die organischen Phasen werden vereinigt, getrocknet und eingeengt. Der Einengungsrückstand wird aus Isopropanol umkristallisiert.

Ausbeute: 95 g (77,8 % der Theorie),
Schmelzpunkt: 175 - 176° C

b) 4-(Phthalimido)-cyclohexanon
95 g (0,388 Mol) 4-(Phthalimido)-cyclohexanol werden in 600 ml Chloroform gelöst und nach Zugabe von 450 ml Wasser und 120 ml Schwefelsäure portionsweise mit 90 g (0,3 Mol) Kaliumdichromat versetzt. Die Innentemperatur des Ansatzes wird hierbei durch leichte Kühlung zwischen 25 und 30° C gehalten. Man rührt 3 Stunden nach, trennt dann die Chloroformphase ab und extrahiert noch zweimal mit Chloroform. Nach dem Trocknen und Einengen der Extrakte erhält man 82 g (86,9 % der Theorie).

c) 2-Amino-6-phthalimido-4,5,6,7-tetrahydro-benzthiazol
48,6 g (0,2 Mol) 4-(Phthalimido)-cyclohexanon werden analog Beispiel 1 mit 32 g (0,2 Mol) Brom bromiert und anschließend mit Thioharnstoff in das 2-Amino-6-phthalimido-4,5,6,7-tetrahydro-benzthiazol übergeführt.

Ausbeute: 30 g (50 % der Theorie),
Schmelzpunkt: 244 - 246° C (Zersetzung)

| | | | |
|---|---|---|---|
| Ber.: | C 60,18 | H 4,38 | N 14,04 |
| Gef.: | 60,05 | 4,25 | 13,95 |

d) 2,6-Diamino-4,5,6,7-tetrahydro-benzthiazol-dihydrochlorid
9,5 g (31,7 mMol) 2-Amino-6-phthalimido-4,5,6,7-tetrahydro-benzthiazol werden in 100 ml Ethanol suspendiert und nach Zugabe von 1,8 g (36 mMol) Hydrazinhydrat 2 Stunden am Rückfluß gekocht. Anschließend wird eingeengt und im Fließmittel Methanol an Kieselgel säulenchromatographisch gereinigt. Dann wird in Ethanol mit ethanolischer Salzsäure das Dihydrochlorid gefällt.
Ausbeute: 2,0 g (26 % der Theorie),
Schmelzpunkt: > 315° C (Zersetzung)

| | | | | |
|---|---|---|---|---|
| Ber.: | C 34,72 | H 5,41 | N 17,35 | Cl 29,25 |
| Gef.: | 35,00 | 5,26 | 16,95 | 29,10 |

**Beispiel 3**

6-Acetylamino-2-amino-4,5,6,7-tetrahydro-benzthiazol-hydrobromid

Zu einer Lösung von 155 g (1,0 Mol) 4-Acetylamino-cyclohexanon in 1,5 l Eisessig werden 160 g (1,0 Mol) Brom getropft. Bei Raumtemperatur wird 3 Stunden gerührt. Zu dem Reaktionsgemisch werden 152,0 g (2,0 Mol) Thioharnstoff gegeben und 30 Minuten unter Rückfluß erhitzt. Nach dem Abkühlen werden die ausgefallenen Kristalle abgesaugt und mit Wasser und Aceton gewaschen.

Ausbeute: 73 g (37 % der Theorie),
Schmelzpunkt: 292 - 293° C (Zersetzung)

| | | | |
|---|---|---|---|
| Ber.: | C 36,99 | H 4,83 | N 14,38 |
| Gef.: | 36,82 | 4,76 | 14,18 |

Durch Rühren des Hydrobromids in wässriger Kaliumcarbonatlösung und anschließendem Absaugen erhält man die freie Base vom Schmelzpunkt 194 - 196° C (Methanol).

Analog Beispiel 3 wurden folgende Verbindungen hergestellt:

6-Acetylamino-2-allylamino-4,5,6,7-tetrahydro-benzthiazol
Ausbeute: 46 % der Theorie,
Schmelzpunkt: 194 - 196° C
Ber.: m/e = 251
Gef.: m/e = 251

6-Acetylamino-2-methylamino-4,5,6,7-tetrahydro-benzthiazol
Ausbeute: 64 % der Theorie,
Schmelzpunkt: 238 - 240° C

| | | | |
|---|---|---|---|
| Ber.: | C 53,30 | H 6,71 | N 18,65 |
| Gef.: | 53,18 | 6,78 | 18,41 |

6-Acetylamino-2-dimethylamino-4,5,6,7-tetrahydro-benzthiazol
Ausbeute: 51 % der Theorie,
Schmelzpunkt: 170 - 171° C

| | | | |
|---|---|---|---|
| Ber.: | C 55,20 | H 7,16 | N 17,56 |
| Gef.: | 55,15 | 7,17 | 17,58 |

**Beispiel 4**

2,6-Diamino-4,5,6,7-tetrahydro-benzthiazol-dihydrobromid

3 g (0,01 Mol) 6-Acetylamino-2-amino-4,5,6,7-tetrahydro-benzthiazol-hydrobromid werden in 20 ml halbkonzentrierter Bromwasserstoffsäure gelöst und 6 Stunden unter Rückfluß erhitzt. Anschließend wird die Lösung eingeengt und der Rückstand aus Methanol umkristallisiert.
Ausbeute: 2,8 g (82 % der Theorie),
Schmelzpunkt: > 315° C,
Schmelzpunkt der Base: 233 - 236° C

| | | | |
|---|---|---|---|
| Ber.: | C 25,39 | H 3,96 | N 12,69 |
| Gef.: | 25,34 | 3,93 | 12,51 |

Analog Beispiel 4 wurden folgende Verbindungen hergestellt:

6-Amino-2-methylamino-4,5,6,7-tetrahydro-benzthiazol-hydrobromid
Ausbeute: 57 % der Theorie,
Schmelzpunkt: 262 - 263° C

| | | | |
|---|---|---|---|
| Ber.: | C 36,37 | H 5,34 | N 15,90 |
| Gef.: | 36,30 | 5,45 | 15,82 |

2-Allylamino-6-amino-4,5,6,7-tetrahydro-benzthiazol-oxalat
Ausbeute: 52 % der Theorie,

Schmelzpunkt: 164 - 165° C (Zersetzung)
Ber.: m/e = 209
Gef.: m/e = 209

6-Amino-2-dimethylamino-4,5,6,7-tetrahydro-benzthiazol-dihydrobromid
Ausbeute: 45 % der Theorie,
Schmelzpunkt: > 270° C (Zersetzung)

| | | | |
|---|---|---|---|
| Ber.: | C 30,10 | H 4,77 | N 11,70 |
| Gef.: | 30,13 | 4,84 | 11,68 |

**Beispiel 5**

2-Amino-6-[N-(2-phenyl-ethyl)-amino]-4,5,6,7-tetrahydro-benzthiazol-dihydrochlorid
Zu einer Lösung aus 3,4 g (0,02 Mol) 2,6-Diamino-tetrahydro-benzthiazol in 34 ml Dimethylformamid werden 5 g (0,022 Mol) 2-Phenyl-ethylbromid und 2,6 g Kaliumkarbonat gegeben und die Reaktionsmischung bei 100° C 3 Stunden gerührt. Anschließend wird das ausgefallene Kaliumbromid abgesaugt und das Lösungsmittel abdestilliert. Der Rückstand wird an Kieselgel chromatographiert (Essigsäureethylester/Methanol = 80/20 + 3 % Ammoniak). Durch Zugabe etherischer Salzsäure kristallisiert die gewünschte Verbindung.
Ausbeute: 2,1 g (30 % der Theorie),
Schmelzpunkt: 289 - 291° C

| | | | |
|---|---|---|---|
| Ber.: | C 52,02 | H 6,11 | N 12,13 |
| Gef.: | 51,82 | 6,13 | 12,16 |

Analog Beispiel 5 wurden folgende Verbindungen hergestellt:

2-Amino-6-isopropylamino-4,5,6,7-tetrahydro-benzthiazol-dihydrochlorid
Ausbeute: 28 % der Theorie,
Schmelzpunkt: 295 - 296° C (Zersetzung)

| | | | |
|---|---|---|---|
| Ber.: | C 42,25 | H 6,74 | N 14,78 |
| Gef.: | 41,95 | 7,09 | 14,50 |

2-Amino-6-isobutylamino-4,5,6,7-tetrahydro-benzthiazol-dihydrochlorid
Ausbeute: 35 % der Theorie,
Schmelzpunkt: 268° C (Zersetzung)

| | | | |
|---|---|---|---|
| Ber.: | C 44,29 | H 7,10 | N 14,09 |
| Gef.: | 43,97 | 7,17 | 13,97 |

6-Allylamino-2-amino-4,5,6,7-tetrahydro-benzthiazol-dihydrochlorid
Ausbeute: 38 % der Theorie,
Schmelzpunkt: 282 - 283° C (Zersetzung)

| | | | |
|---|---|---|---|
| Ber.: | C 42,56 | H 6,07 | N 14,89 |
| Gef.: | 42,17 | 6,07 | 14,71 |

2-Amino-6-[N-(2-chlor-benzyl)-amino]-4,5,6,7-tetrahydro-benzthiazol-dihydrochlorid
Ausbeute: 40 % der Theorie,
Schmelzpunkt: > 280° C (Zersetzung)

| | | | |
|---|---|---|---|
| Ber.: | C 45,85 | H 4,95 | N 11,45 |
| Gef.: | 45,50 | 4,86 | 11,08 |

2-Amino-6-propargylamino-4,5,6,7-tetrahydro-benzthiazol-dihydrochlorid
Ausbeute: 35 % der Theorie,
Schmelzpunkt: 268 - 270° C (Zersetzung)

| | | | |
|---|---|---|---|
| Ber.: | C 42,86 | H 5,40 | N 15,00 |
| Gef.: | 42,78 | 5,59 | 14,79 |

2-Amino-6-methylamino-4,5,6,7-tetrahydro-benzthiazol-dihydrobromid

Ausbeute: 25 % der Theorie,
Schmelzpunkt: 312 - 313° C (Zersetzung)

| | | | |
|---|---|---|---|
| Ber.: | C 27,84 | H 4,38 | N 12,18 |
| Gef.: | 27,78 | 4,46 | 12,21 |

**Beispiel 6**

2-Amino-6-di-n-propylamino-4,5,6,7-tetrahydro-benzthiazol-dihydrochlorid-monohydrat

Zu einer Lösung von 3,4 g (0,02 Mol) 2,6-Diamino-4,5,6,7-tetrahydro-benzthiazol in 50 ml Methanol werden 10 g (0,08 Mol) n-Propylbromid und 11,1 g Kaliumkarbonat gegeben und 3 Tage am Rückfluß erhitzt. Anschließend wird mit 100 ml Wasser versetzt und mit Essigsäureethylester extrahiert. Das Lösungsmittel wird abdestilliert und der Rückstand an Kieselgel (Fließmittel: Methylenchlorid/Methanol = 80/20) chromatographiert. Die entsprechende Fraktion wird eingeengt und die gewünschte Verbindung als Hydrochlorid gefällt.
Ausbeute: 1,9 g (28 % der Theorie),
Schmelzpunkt: 271 - 273° C

| | | | |
|---|---|---|---|
| Ber.: | C 45,34 | H 7,90 | N 12,20 |
| Gef.: | 45,00 | 7,98 | 12,00 |

**Beispiel 7**

2-Amino-6-n-butylamino-4,5,6,7-tetrahydro-benzthiazol-dihydrochlorid

Zu einer Lösung aus 3,4 g (0,02 Mol) 2,6-Diamino-4,5,6,7-tetrahydro-benzthiazol in 34 ml Dimethylformamid werden 1,8 g (0,022 Mol) n-Butanal gegeben und 1 Stunde auf 50° C erwärmt. Nach dem Abkühlen wird die Reaktionslösung mit 0,8 g (0,02 Mol) Natriumborhydrid versetzt und 30 Minuten auf 50° C erwärmt. Das Lösungsmittel wird im Vakuum weitgehend entfernt. Der Rückstand wird unter Eiskühlung mit 20 ml Wasser und 2N Salzsäure bis pH = 1 versetzt. Die wäßrige Lösung wird mit Essigsäureethylester extrahiert. Die organische Phase wird verworfen. Die wässrige Phase wird mit Kaliumcarbonat bis zur alkalischen Reaktion versetzt und mit Essigsäureethylester extrahiert. Die organische Phase wird getrocknet und eingeengt. Unter Zusatz von etherischer Salzsäure kristallisiert die Verbindung.
Ausbeute: 2,3 g (39 % der Theorie),
Schmelzpunkt: 254 - 256° C

| | | | |
|---|---|---|---|
| Ber.: | C 44,29 | H 7,10 | N 14,09 |
| Gef.: | 44,44 | 7,31 | 14,07 |

Analog Beispiel 7 wurden nachfolgende Verbindungen hergestellt:

2-Amino-6-ethylamino-4,5,6,7-tetrahydro-benzthiazol-dihydrochlorid
Ausbeute: 38 % der Theorie,
Schmelzpunkt: 296 - 297° C

| | | | |
|---|---|---|---|
| Ber.: | C 40,00 | H 6,34 | N 15,55 |
| Gef.: | 39,97 | 6,41 | 15,35 |

2-Amino-6-n-pentylamino-4,5,6,7-tetrahydro-benzthiazol-semi-fumarat
Ausbeute: 42 % der Theorie,
Schmelzpunkt: > 270° C

| | | | |
|---|---|---|---|
| Ber.: | C 56,54 | H 7,79 | N 14,13 |
| Gef.: | 56,13 | 7,80 | 13,97 |

2-Amino-6-n-hexylamino-4,5,6,7-tetrahydro-benzthiazol-dihydrochlorid
Ausbeute: 49 % der Theorie,
Schmelzpunkt: 272 - 274° C

| | | | |
|---|---|---|---|
| Ber: | C 47,85 | H 7,72 | N 12,88 |
| Gef: | 47,96 | 7,65 | 12,71 |

2-Amino-6-n-propylamino-4,5,6,7-tetrahydro-benzthiazol-dihydrochlorid

Ausbeute: 42 % der Theorie, Schmelzpunkt: 286 - 288° C

| | | | |
|---|---|---|---|
| Ber.: | C 42,25 | H 6,74 | N 14,78 |
| Gef.: | 42,05 | 6,77 | 14,57 |

(-) 2-Amino-6-n-propylamino-4,5,6,7-tetrahydro-benzthiazol-dihydrochlorid
Schmelzpunkt: 270 - 272° C
$\alpha^{20}_D$ = -56° (c = I, Methanol)

(+) 2-Amino-6-n-propylamino-4,5,6,7-tetrahydro-benzthiazol-dihydrochlorid
Schmelzpunkt: 270 - 272° C
$\alpha^{20}_D$ = +56° (c = I, Methanol)

2-Amino-6-cyclopentylamino-4,5,6,7-tetrahydro-benzthiazol-dioxalat
Ausbeute: 36 % der Theorie,
Schmelzpunkt: 212 - 213° C

| | | | |
|---|---|---|---|
| Ber.: | C 46,04 | H 5,55 | N 10,07 |
| Gef.: | 45,95 | 5,28 | 10,08 |

2-Amino-6-cyclohexylamino-4,5,6,7-tetrahydro-benzthiazol-dihydrochlorid
Ausbeute: 38 % der Theorie,
Schmelzpunkt: 288 - 290° C

| | | | |
|---|---|---|---|
| Ber.: | C 48,14 | H 7,15 | N 12,96 |
| Gef.: | 47,88 | 7,16 | 12,74 |

**Beispiel 8**

6-Ethylamino-2-methylamino-4,5,6,7-tetrahydro-benzthiazol-dihydrochlorid
Eine Lösung aus 1 g (0,0044 Mol) 6-Acetylamino-2-methylamino-4,5,6,7-tetrahydro-benzthiazol in 20 ml absolutem Tetrahydrofuran wird mit 0,4 g (0,01 Mol) Lithiumaluminiumhydrid versetzt und 2 Stunden am Rückfluß erhitzt. Nach dem Abkühlen werden 50 g einer 40-%-igen Diammoniumtartratlösung zugetropft. Die organische Phase wird abgetrennt und eingeengt. Der Rückstand wird an Kieselgel chromatographiert (Fließmittel: Methylenchlorid/Methanol = 80/20). Die entsprechende Fraktion wird eingeengt. Unter Zugabe von etherischer Salzsäure kristallisiert die Verbindung.
Ausbeute: 0,3 g (33 % der Theorie),
Schmelzpunkt: > 260° C;
Ber.: m/e = 211
Gef.: m/e = 211

Analog Beispiel 8 wurden die folgenden Verbindungen hergestellt:

2-Allylamino-6-ethylamino-4,5,6,7-tetrahydro-benzthiazol-dihydrochlorid
Ausbeute: 37 % der Theorie,
Schmelzpunkt: 218 - 220° C (Zersetzung)

| | | | |
|---|---|---|---|
| Ber.: | C 46,45 | H 6,82 | N 13,54 |
| Gef.: | 46,60 | 7,03 | 13,66 |

2-Dimethylamino-6-ethylamino-4,5,6,7-tetrahydro-benzthiazol-oxalat-hydrat
Ausbeute: 20 % der Theorie,
Schmelzpunkt: 189 - 190° C

| | | | |
|---|---|---|---|
| Ber.: | C 46,83 | H 6,95 | N 12,60 |
| Gef.: | 47,03 | 6,89 | 12,49 |

**Beispiel 9**

6-Acetylamino-2-benzoylamino-4,5,6,7-tetrahydro-benzthiazol
Zu einer Lösung aus 4,2 g (0,02 Mol) 6-Acetylamino-2-amino-4,5,6,7-tetrahydro-benzthiazol in 100 ml

absolutem Tetrahydrofuran werden 2,2 g (0,022 Mol) Triethylamin und 3,1 g (0,022 Mol) Benzoylchlorid gegeben und 3 Stunden unter Rückfluß erhitzt. Die Reaktionsmischung wird mit Wasser versetzt und mit Essigsäureethylester extrahiert. Die organische Phase wird eingeengt. Der Rückstand wird aus Methanol umkristallisiert.
Ausbeute: 3 g (48 % der Theorie),
Schmelzpunkt: > 260° C
Ber.: m/e = 315
Gef.: m/e = 315

Analog Beispiel 9 wurden die folgenden Verbindungen hergestellt:

2,6-Diacetylamino-4,5,6,7-tetrahydro-benzthiazol
Ausbeute: 50 % der Theorie,
Schmelzpunkt: 258 - 259° C
Ber.: m/e = 252
Gef.: m/e = 252

6-Acetylamino-2-propionylamino-4,5,6,7-tetrahydro-benzthiazol
Ausbeute: 44 % der Theorie,
Schmelzpunkt: > 260° C
Ber.: m/e = 266
Gef.: m/e = 266

6-Acetylamino-2-phenylacetylamino-4,5,6,7-tetrahydro-benzthiazol
Ausbeute: 78 % der Theorie,
Schmelzpunkt: 112° C
Ber.: m/e = 329
Gef.: m/e = 329

**Beispiel 10**

2-Benzylamino-6-ethylamino-4,5,6,7-tetrahydro-benzthiazol-dihydrochlorid
Zu einer Lösung aus 1,2 g (3,2 mMol) 6-Acetylamino-2-benzoylamino-4,5,6,7-tetrahydro-benzthiazol in 50 ml absolutem Tetrahydrofuran werden 0,24 g (64 mMol) Lithiumaluminiumhydrid gegeben und 1 Stunde unter Rückfluß erhitzt. Die Aufarbeitung erfolgt analog Beispiel 8.
Ausbeute: 0,4 g (34 % der Theorie),
Schmelzpunkt: 242 - 245° C

| | | | |
|---|---|---|---|
| Ber.: | C 53,33 | H 6,43 | N 19,68 |
| Gef.: | 53,59 | 6,37 | 19,42 |

Analog Beispiel 10 wurden folgende Verbindungen hergestellt:

2,6-Diethylamino-4,5,6,7-tetrahydro-benzthiazol-dihydrochlorid
Ausbeute: 38 % der Theorie,
Schmelzpunkt: 241 - 243° C

| | | | |
|---|---|---|---|
| Ber.: | C 44,29 | H 7,10 | N 14,09 |
| Gef.: | 44,06 | 7,27 | 13,85 |

6-Ethylamino-2-n-propylamino-4,5,6,7-tetrahydro-benzthiazol-dihydrochlorid
Ausbeute: 32 % der Theorie,
Schmelzpunkt: 267 - 268° C

| | | | |
|---|---|---|---|
| Ber.: | C 46,15 | H 7,42 | N 13,46 |
| Gef.: | 45,95 | 7,53 | 13,33 |

6-Ethylamino-2-[N-(2-phenyl-ethyl)-amino]-4,5,6,7-tetrahydro-benzthiazol-dihydrochlorid-halbhydrat
Ausbeute: 26 % der Theorie,
Schmelzpunkt: 248 - 251° C

| | | | |
|---|---|---|---|
| Ber.: | C 53,25 | H 6,84 | N 10,96 |
| Gef.: | 53,3 | 6,64 | 10,89 |

2-(4-Chlor-benzylamino)-6-ethylamino-4,5,6,7-tetrahydro-benzthiazol-dihydrochlorid
Ausbeute: 65 % der Theorie,
Schmelzpunkt: > 260° C

| | | | |
|---|---|---|---|
| Ber.: | C 48,67 | H 5,62 | N 10,64 |
| Gef.: | 48,79 | 5,80 | 10,60 |

2-(2-Chlor-benzylamino)-6-ethylamino-4,5,6,7-tetrahydro-benzthiazol-dihydrochlorid
Ausbeute: 36 % der Theorie,
Schmelzpunkt: 251 - 253° C

| | | | |
|---|---|---|---|
| Ber.: | C 48,67 | H 5,62 | N 10,64 |
| Gef.: | 48,57 | 5,78 | 10,57 |

2-(3,4-Dichlor-benzylamino)-6-ethylamino-4,5,6,7-tetrahydro-benzthiazol-dihydrochlorid
Ausbeute: 62,5 % der Theorie,
Schmelzpunkt: > 260° C

| | | | |
|---|---|---|---|
| Ber.: | C 44,77 | H 4,93 | N 9,79 |
| Gef.: | 44,85 | 4,82 | 9,96 |

6-Acetylamino-2-ethylamino-4,5,6,7-tetrahydro-benzthiazol
Hergestellt aus 2,6-Diacetylamino-4,5,6,7-tetrahydro-benzthiazol bei Raumtemperatur.
Ausbeute: 33 % der Theorie,
Schmelzpunkt: 234 - 235° C
Ber.: m/e = 238
Gef.: m/e = 238

6-Acetylamino-2-benzylamino-4,5,6,7-tetrahydro-benzthiazol

Hergestellt aus 6-Acetylamino-2-benzoylamino-4,5,6,7-tetrahydro-benzthiazol bei Raumtemperatur.

6-Acetylamino-2-n-propylamino-4,5,6,7-tetrahydro-benzthiazol

Hergestellt aus 6-Acetylamino-2-propionylamino-4,5,6,7-tetrahydro-benzthiazol bei Raumtemperatur.

6-Acetylamino-2-[N-(2-phenyl-ethyl)-amino]-4,5,6,7-tetrahydro-benzthiazol

**Beispiel 11**

6-Amino-2-ethylamino-4,5,6,7-tetrahydro-benzthiazol-dihydrochlorid
Hergestellt aus 6-Acetylamino-2-ethylamino-4,5,6,7-tetrahydro-benzthiazol analog Beispiel 4.
Ausbeute: 45 % der Theorie,
Schmelzpunkt: 155 - 158° C

| | | | |
|---|---|---|---|
| Ber.: | C 40,00 | H 6,34 | N 15,55 |
| Gef.: | 39,86 | 6,31 | 15,26 |

Analog Beispiel 11 wurden folgende Verbindungen hergestellt:

6-Amino-2-benzylamino-4,5,6,7-tetrahydro-benzthiazol-dihydrobromid

6-Amino-2-n-propylamino-4,5,6,7-tetrahydro-benzthiazol-dihydrobromid

6-Amino-2-[N-(2-phenyl-ethyl)amino]-4,5,6,7-tetrahydro-benzthiazol-dihydrobromid

**Beispiel 12**

2-Benzoylamino-6-dimethylamino-4,5,6,7-tetrahydro-benzthiazol-dihydrochlorid
3,0 g (15 mMol) 2-Amino-6-dimethylamino-4,5,6,7-terrahydro-benzthiazol werden in 15 ml Pyridin gelöst und tropfenweise mit 2,1 g (15 mMol) Benzoylchlorid versetzt. Nach Stehen über Nacht engt man ein, versetzt mit

Sodalösung und extrahiert mit Chloroform. Den Chloroformextrakt engt man ein und chromatographiert anschließend an Kieselgel (Fließmittel: Methylenchlorid/Methanol = 9/1). Die isolierte Base (Schmelzpunkt: 174° C) wird in Aceton gelöst und mit isopropanolischer Salzsäure das Dihydrochlorid gefällt.

Ausbeute: 2,8 g (49 % der Theorie),
Schmelzpunkt: 284° C (Zersetzung)

| | | | | |
|---|---|---|---|---|
| Ber.: | C 51,33 | H 5,65 | N 11,23 | Cl 18,94 |
| Gef.: | 51,51 | 5,76 | 11,32 | 18,75 |

**Beispiel 13**

6-Acetylamino-2-amino-4,5,6,7-tetrahydro-benzthiazol

3,1 g (20 mMol) 4-Acetylamino-cyclohexanon und 6,2 g (20 mMol) Formamidin-disulfid-dihydrobromid werden innig vermischt und im Heizbad bei einer Temperatur von 120 - 130° C 2 Stunden unter Rühren erhitzt. Anschließend nimmt man mit Wasser auf, stellt mit Ammoniak alkalisch und extrahiert mit Chloroform. Nach dem Trocknen der Extrakte wird eingeengt, mit Aceton digeriert und abgesaugt.

Ausbeute: 1,8 g (42,6 % der Theorie),
Schmelzpunkt: 195° C (Zersetzung)

| | | | |
|---|---|---|---|
| Ber.: | C 51,17 | H 6,20 | N 19,89 |
| Gef.: | 51,09 | 6,22 | 19,75 |

Analog Beispiel 13 wurde ausgehend von 4-Dimethylamino-cyclohexanon nachstehende Verbindung hergestellt:

2-Amino-6-dimethylamino-4,5,6,7-tetrahydro-benzthiazol

Ausbeute: 21 % der Theorie,
Schmelzpunkt: 189 - 190° C

| | | | |
|---|---|---|---|
| Ber.: | C 54,80 | H 7,66 | N 21,29 |
| Gef.: | 54,71 | 7,53 | 21,12 |

**Beispiel I**

Dragéekern mit 5 mg 2-Amino-6-dimethylamino-4,5,6,7-tetrahydro-benzthiazol-dihydrochlorid

Zusammensetzung:
1 Dragéekern enthält:

| | |
|---|---|
| Wirksubstanz | 5,0 mg |
| Milchzucker | 33,5 mg |
| Maisstärke | 10,0 mg |
| Gelatine | 1,0 mg |
| Magnesiumstearat | 0,5 mg |
| | 50,0 mg |

Herstellungsverfahren

Die Mischung der Wirksubstanz mit Milchzucker und Maisstärke wird mit einer 10 %-igen wässrigen Gelatinelösung durch ein Sieb von 1 mm Maschenweite granuliert, bei 40° C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

| | |
|---|---|
| Kerngewicht: | 50 mg |
| Stempel: | 5 mm, gewölbt |

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 100 mg

**Beispiel II**

Tropfen mit 5 mg 2-Amino-6-dimethylamino-4,5,6,7-tetrahydro-benzthiazol-dihydrochlorid

Zusammensetzung:
100 ml Tropfsubstanz enthalten:

| | | |
|---|---|---|
| p-Hydroxybenzoesäure-methylester | 0,035 | g |
| p-Hydroxybenzoesäure-n-propylester | 0,015 | g |
| Anisöl | 0,05 | g |
| Menthol | 0,06 | g |
| Ethanol rein | 10,0 | g |
| Wirksubstanz | 0,5 | g |
| Zitronensäure | 0,7 | g |
| Natriumphosphat sek. x 2 $H_2O$ | 0,3 | g |
| Natriumcyclamat | 1,0 | g |
| Glycerin | 15,0 | g |
| Dest. Wasser | ad 100,0 | ml |

Herstellungsverfahren:
Die p-Hydroxybenzoesäureester, Anisöl sowie Menthol werden in Ethanol gelöst (Lösung I).
Die Puffersubstanzen, die Wirksubstanz und Natriumcyclamat werden in dest. Wasser gelöst und Glycerin zugefügt (Lösung II). Lösung I wird in Lösung II eingerührt und die Mischung mit dest. Wasser auf das gegebene Volumen aufgefüllt. Die fertige Tropflösung wird durch ein geeignetes Filter filtriert. Die Herstellung und Abfüllung der Tropflösung muß unter Lichtschutz und unter Schutzbegasung erfolgen.

**Beispiel III**

Suppositorien mit 10 mg 2-Amino-6-dimethylamino-4,5,6,7-tetrahydro-benzthiazol-dihydrochlorid

1 Zäpfchen enthält:

| | |
|---|---|
| Wirksubstanz | 10,0 mg |
| Zäpfchenmassen (z. B. Witepsol W 45) | 1 690,0 mg |
| | 1 700,0 mg |

Herstellungsverfahren:
Die feingepulverte Substanz wird mit Hilfe eines Eintauchhomogenisators in die geschmolzene und auf 40° C abgekühlte Zäpfchenmasse eingerührt. Die Masse wird bei 35° C in leicht vorgekühlte Formen ausgegossen.

Zäpfchengewicht: 1,7 g

**Beispiel IV.**

Ampullen mit 5 mg 2-Amino-6-dimethylamino-4,5,6,7-tetrahydro-benzthiazol-dihydrochlorid

1 Ampulle enthält:

| | |
|---|---|
| Wirksubstanz | 5,0 mg |
| Zitronensäure | 7,0 mg |
| Natriumphosphat sek. x 2 $H_2O$ | 3,0 mg |
| Natriumpyrosulfit | 1,0 mg |
| Dest. Wasser | ad 1,0 ml |

Herstellungsverfahren:
In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.

| | |
|---|---|
| Abfüllung: | in braune Ampullen unter Schutzbegasung |
| Sterilisation: | 20 Minuten bei 120° C |

Die Herstellung und Abfüllung der Ampullenlösung muß in abgedunkelten Räumen vorgenommen werden.

**Beispiel V**

Dragées mit 1 mg 2-Amino-6-dimethylamino-4,5,6,7-tetrahydro-benzthiazol-dihydrochlorid
>r

1 Dragéekern enthält:

| | |
|---|---|
| Wirksubstanz | 1,0 mg |
| Milchzucker | 35,5 mg |
| Maisstärke | 12,0 mg |
| Gelatine | 1,0 mg |
| Magnesiumstearat | 0,5 mg |
| | 50,0 mg |

Herstellungsverfahren:
Analog Beispiel I.

| | |
|---|---|
| Kerngewicht: | 50 mg |
| Stempel: | 5 mm, gewölbt |
| Dragéegewicht: | 100 mg |

Als Wirkstoffe können in die pharmazeutischen Anwendungsbeispiele I bis V selbstverständlich an Stelle der genannten Verbindung alle übrigen Verbindungen der allgemeinen Formel I eingearbeitet werden wie z. B. 2-Amino-6-n-propylamino-4,5,6,7-tetrahydro-benzthiazol-dihydrochlorid.

**Patentansprüche**

1. Tetrahydro-benzthiazole der allgemeinen Formel

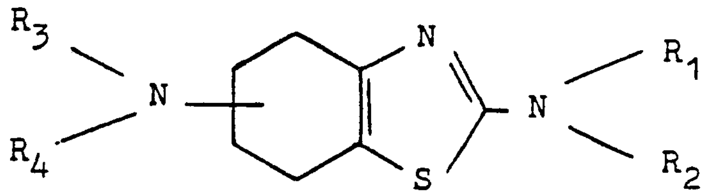

,(I)

in der

$R_1$ ein Wasserstoffatom, eine Alkylgruppe mit 1 bis 6 Kohlenstoffatomen, eine Alkenyl- oder Alkinylgruppe mit jeweils 3 bis 6 Kohlenstoffatomen, eine Alkanoylgruppe mit 1 bis 6 Kohlenstoffatomen, eine Phenylalkyl- oder Phenylalkanoylgruppe mit jeweils 1 bis 3 Kohlenstoffatomen im Alkylteil, wobei die oben erwähnten Phenylkerne jeweils durch 1 oder 2 Halogenatome substituiert sein können,

$R_2$ ein Wasserstoffatom oder eine Alkylgruppe mit 1 bis 4 Kohlenstoffatomen,

$R_3$ ein Wasserstoffatom, eine Alkylgruppe mit 1 bis 7 Kohlenstoffatomen, eine Cycloalkylgruppe mit 3 bis 7 Kohlenstoffatomen, eine Alkenyl- oder Alkinylgruppe mit jeweils 3 bis 6 Kohlenstoffatomen, eine Alkanoylgruppe mit 1 bis 7 Kohlenstoffatomen, eine Phenylalkyl- oder Phenylalkanoylgruppe mit jeweils 1 bis 3 Kohlenstoffatomen im Alkylteil, wobei der Phenylkern durch Fluor-, Chlor- oder Bromatome substituiert sein kann,

$R_4$ ein Wasserstoffatom, eine Alkylgruppe mit 1 bis 4 Kohlenstoffatomen, eine Alkenyl- oder Alkinylgruppe mit jeweils 3 bis 6 Kohlenstoffatomen oder

$R_3$ und $R_4$ zusammen mit dem dazwischen liegenden Stickstoffatom eine Pyrrolidino-, Piperidino-, Hexamethylenimino- oder Morpholinogruppe bedeuten, deren Enantiomere und deren Säureadditionssalze.

2. Tetrahydro-benzthiazole der allgemeinen Formel I gemäß Anspruch 1, in der die

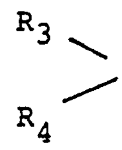

N-Gruppe in 5- oder 6-Stellung steht, deren Enantiomere und deren Säureadditionssalze.

3. Tetrahydro-benzthiazole der allgemeinen Formel

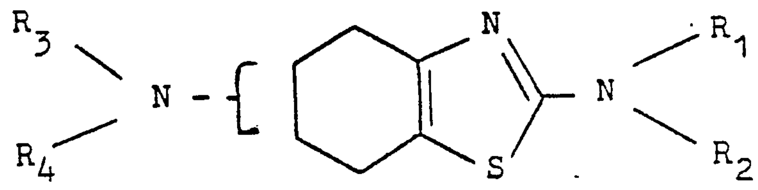 ,(Ia)

in der

$R_1$ ein Wasserstoffatom, eine Alkylgruppe mit 1 bis 3 Kohlenstoffatomen, eine Allyl-, Benzyl-, 2-Chlor-benzyl-, 4-Chlor-benzyl-, 3,4-Dichlor-benzyl- oder Phenylethylgruppe,

$R_2$ ein Wasserstoffatom, eine Methyl- oder Ethylgruppe,

$R_3$ ein Wasserstoffatom, eine Alkylgruppe mit 1 bis 6 Kohlenstoffatomen, eine Allyl-, Propargyl-, Benzyl-, Chlorbenzyl-, Phenylethyl-, Cyclopentyl- oder Cyclohexylgruppe,

$R_4$ ein Wasserstoffatom, eine Alkylgruppe mit 1 bis 3 Kohlenstoffatomen oder eine Allylgruppe oder

$R_3$ und $R_4$ zusammen mit dem dazwischen liegenden Stickstoffatom eine Pyrrolidino-, Piperidino-, Hexamethylenimino- oder Morpholinogruppe bedeuten, deren Enantiomere und deren Säureadditionssalze.

4. Tetrahydro-benzthiazole der allgemeinen Formel Ia gemäß Anspruch 3, in der die

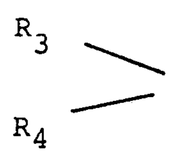

N-Gruppe in 6-Stellung steht, deren Enantiomere und deren Säureadditionssalze.

5. Tetrahydro-benzthiazole der allgemeinen Formel Ia gemäß Anspruch 3, in der

$R_1$ und $R_2$ zusammen mit dem dazwischenliegenden Stickstoffatom eine Amino- oder Allylaminogruppe und

$R_3$ und $R_4$ zusammen mit den dazwischenliegenden Stickstoffatom eine Dimethylamino-, Diethylamino-, N-Allyl-N-(4-chlor-benzyl)-amino-, n-Propylamino- oder Pyrrolidinogruppe darstellen, deren Enantiomere und deren Säureadditionssalze.

6. 2-Amino-6-n-propylamino-4,5,6,7-tetrahydro-benzthiazol, dessen Enantiomere und dessen Säureadditionssalze.

7. (-)-2-Amino-6-n-propylamino-4,5,6,7-tetrahydro-benzthiazol und dessen Säureadditionssalze.

8. Physiologisch verträgliche Säureadditionssalze mit anorganischen oder organischen Säuren der Verbindungen gemäß den Ansprüchen 1 bis 7.

9. Arzneimittel, enthaltend als Wirkstoff eine Verbindung gemäß den Ansprüchen 3 bis 7 oder ein physiologisch verträgliches Säureadditionssalz gemäß Anspruch 8 neben gegebenenfalls einem oder mehreren inerten Trägerstoffen und/oder Verdünnungsmitteln.

10. Verwendung einer Verbindung gemäß den Ansprüchen 3 bis 7 oder eines physiologisch verträglichen Säureadditionssalzes gemäß Anspruch 8 zur Herstellung eines Arzneimittels, welches zur Behandlung von zentralnervösen neuropsychiatrischen Erkrankungen und/oder zur Behandlung von Kreislauferkrankungen geeignet ist.

11. Verwendung einer Verbindung gemäß den Ansprüchen 3 bis 7 oder eines physiologisch verträglichen Säureadditionssalzes gemäß Anspruch 8 zur Herstellung eines Arzneimittels, welches zur Behandlung der Parkinson'schen-Erkrankung bzw. des Parkinsonismus geeignet ist.

12. Verfahren zur Herstellung eines Arzneimittels, dadurch gekennzeichnet, daß auf nichtchemischem Wege eine Verbindung gemäß den Ansprüchen 3 bis 7 oder ein physiologisch verträgliches Säureadditionssalz gemäß Anspruch 8 in einen oder mehrere inerte Trägerstoffe und/oder Verdünnungmittel eingearbeitet wird.

13. Verfahren zur Herstellung der Tetrahydro-benzthiazole gemäß den Ansprüchen 1 bis 8, dadurch

gekennzeichnet, daß

a) ein Cyclohexanon der allgemeinen Formel

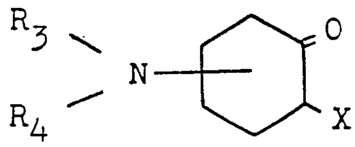

, (II)

in der

$R_3$ und $R_4$ wie in den Ansprüchen 1 bis 7 definiert sind und

X eine nukleophile Austrittsgruppe wie ein Halogenatom, z. B. ein Chlor- oder Bromatom, darstellen, mit einem Thioharnstoff der allgemeinen Formel

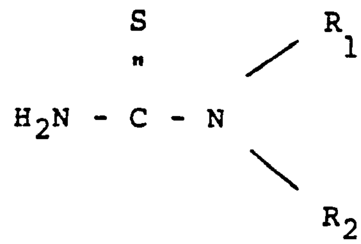

, (III)

in der

$R_1$ und $R_2$ wie in den Ansprüchen 1 bis 7 definiert sind,

umgesetzt wird oder

b) eine Verbindung der allgemeinen Formel

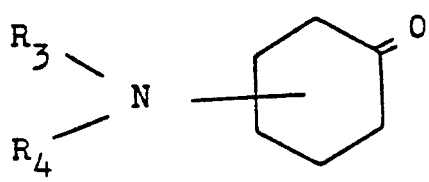

, (IV)

in der

$R_3$ und $R_4$ wie in den Ansprüchen 1 bis 7 definiert sind,

mit einem Formamidindisulfid der allgemeinen Formel

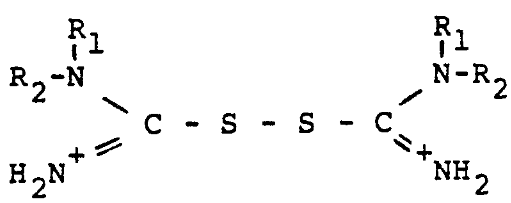

$2\ Y^-$ , (V)

in der

$R_1$ und $R_2$ wie in den Ansprüchen 1 bis 7 definiert sind und

$Y^-$ ein Anion einer anorganischen oder organischen Säure darstellt, umgesetzt wird oder

c) zur Herstellung von Verbindungen der allgemeinen Formel I, in der mindestens einer der Reste $R_1$, $R_2$, $R_3$ oder $R_4$ ein Wasserstoffatom darstellt, ein Schutzrest von einer Verbindung der allgemeinen Formel

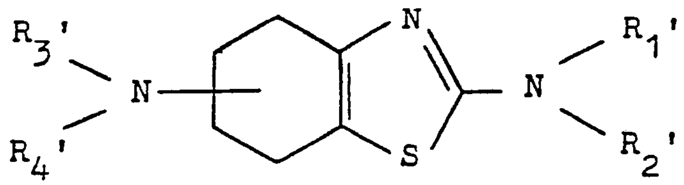

,(VI)

in der

mindestens einer der Reste $R_1'$ $R_2'$, $R_3'$ oder $R_4'$ eine Schutzgruppe für eine Aminogruppe wie eine Acyl- oder Alkoxycarbonylgruppe, z. B. eine Acetyl-, Propionyl-, Methoxycarbonyl- oder Ethoxycarbonylgruppe, oder auch $R_1'$ und $R_2'$ oder $R_3'$ und $R_4'$ zusammen mit dem dazwischen liegenden Stickstoffatom einen Imidorest, z. B. den Phthalimidorest, bedeuten und

die übrigen der Reste $R_1'$, $R_2'$, $R_3'$ oder $R_4'$ die für $R_1$ bis $R_4$ in den Ansprüchen 1 bis 7 erwähnten Bedeutungen mit Ausnahme der in den Ansprüchen 1 und 2 erwähnten Acylreste bedeuten, abgespalten wird oder

d) zur Herstellung von Verbindungen der allgemeinen Formel I, in der mindestens einer der Reste $R_1$, $R_2$, $R_3$ oder $R_4$ eine der in den Ansprüchen 1 bis 7 erwähnten Alkyl- oder Phenylalkylgruppen bedeutet, eine Verbindung der allgemeinen Formel

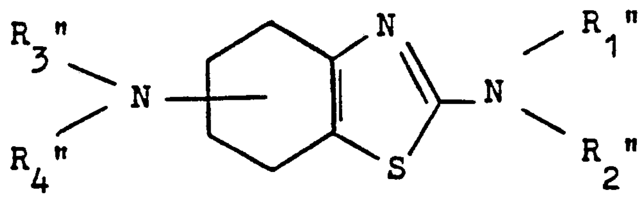

,(VII)

in der

mindestens einer der Reste $R_1''$, $R_2''$, $R_3''$ oder $R_4''$ einen der in den Ansprüchen 1 und 2 erwähnten Acyl- oder Phenylacylreste darstellt und

die übrigen der Reste die für $R_1$, $R_2$, $R_3$ und $R_4$ in den Ansprüchen 1 bis 7 erwähnten Bedeutungen besitzen, mit einem Metallhydrid in einem Lösungsmittel reduziert wird oder

e) zur Herstellung von Verbindungen der allgemeinen Formel I, in der mindestens einer der Reste $R_1$, $R_2$, $R_3$ oder $R_4$ eine der in den Ansprüchen 1 bis 7 erwähnten Alkyl-, Cycloalkyl-, Alkenyl-, Alkinyl- oder Phenylalkylgruppen bedeutet, eine Verbindung der allgemeinen Formel

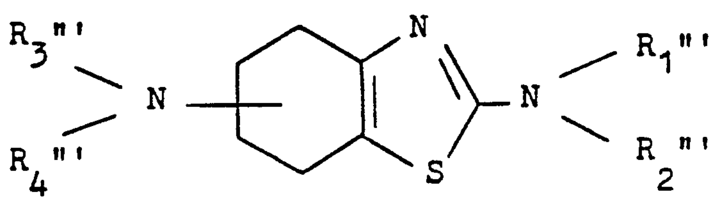

,(VIII)

in der

mindestens einer der Reste $R_1'''$, $R_2'''$, $R_3'''$ oder $R_4'''$ ein Wasserstoffatom darstellt und die übrigen der Reste $R_1'''$, $R_2'''$, $R_3'''$ oder $R_4'''$ die für $R_1$ bis $R_4$ in den Ansprüchen 1 bis 7 erwähnten Bedeutungen besitzen, mit einer Verbindung der allgemeinen Formel

$R_5 - Z$ ,(IX)

in der

$R_5$ eine der für $R_1$ bis $R_4$ in den Ansprüchen 1 bis 7 erwähnten Alkyl-, Cycloalkyl-, Alkenyl-, Alkinyl- oder Phenylalkylgruppen und

Z eine nukleophile Austrittsgruppe wie ein Halogenatom oder einen Sulfonsäurerest, z. B. ein Chlor-, Brom- oder Jodatom, eine Methoxysulfonyloxy- oder p-Toluolsulfonyloxygruppe,

oder Z zusammen mit einem benachbarten Wasserstoffatom des Restes $R_5$ ein Sauerstoffatom bedeuten, umgesetzt wird

und gewünschtenfalls anschließend eine so erhaltene Verbindung der allgemeinen Formel I, in der mindestens einer der Reste $R_1$ oder $R_3$ ein Wasserstoffatom darstellen, mittels entsprechender Acylierung in eine entsprechende Verbindung der allgemeinen Formel I, in der mindestens einer der Reste $R_1$ oder $R_3$ einen der in den Ansprüchen 1 und 2 erwähnten Acylreste darstellt, übergeführt wird oder

eine so erhaltene Verbindung der allgemeinen Formel I, welche mindestens ein chirales Zentrum enthält, in ihre Enantiomeren aufgetrennt wird oder

eine so erhaltene Verbindung der allgemeinen Formel I in ihre Säureadditionssalze, insbesondere in ihre physiologisch verträglichen Säureadditionssalze mit organischen oder anorganischen Säuren übergeführt wird.

## Claims

1. Tetrahydro-benzthiazoles of general formula

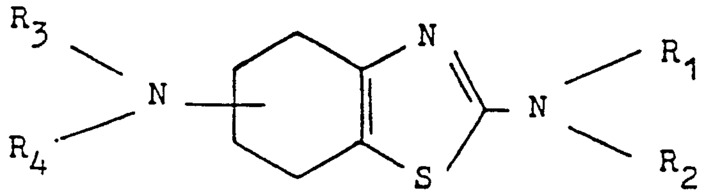

(I)

(wherein

$R_1$ represents a hydrogen atom, an alkyl group having 1 to 6 carbon atoms, an alkenyl or alkynyl group each having 3 to 6 carbon atoms, an alkanoyl group having 1 to 6 carbon atoms, a phenyl alkyl or phenyl alkanoyl group having 1 to 3 carbon atoms in the alkyl part, whilst the above mentioned phenyl nuclei may be substituted by 1 or 2 halogen atoms,

$R_2$ represents a hydrogen atom or an alkyl group with 1 to 4 carbon atoms,

$R_3$ represents a hydrogen atom, an alkyl group with 1 to 7 carbon atoms, a cycloalkyl group having 3 to 7 carbon atoms, an alkenyl or alkynyl group having 3 to 6 carbon atoms, an alkanoyl group having 1 to 7 carbon atoms, a phenyl alkyl or phenyl alkanoyl group having 1 to 3 carbon atoms in the alkyl part, whilst the phenyl nucleus may be substituted by fluorine, chlorine or bromine atoms,

$R_4$ represents a hydrogen atom, an alkyl group with 1 to 4 carbon atoms, an alkenyl or alkynyl group having 3 to 6 carbon atoms or

$R_3$ and $R_4$ together with the nitrogen atom between them represent a pyrrolidino, piperidino, hexamethyleneimino or morpholino group)

the enantiomers thereof, and the acid addition salts thereof.

2. Tetrahydro-benzthiazoles of general formula I as claimed in claim 1 wherein the

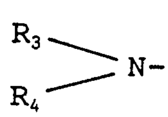

group is in the 5 or 6-position, the enantiomers thereof, and the acid addition salts thereof.

3. Tetrahydro-benzthiazoles of general formula

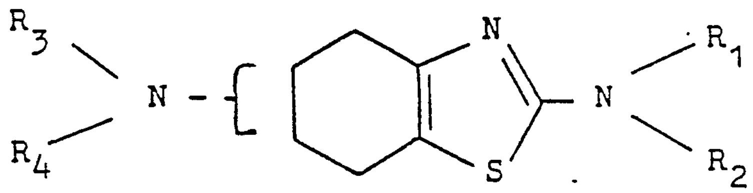

(Ia)

(wherein

$R_1$ represents a hydrogen atom, an alkyl group having 1 to 3 carbon atoms, an allyl, benzyl, 2-chloro-benzyl, 4-chloro-benzyl, 3,4-dichloro-benzyl or phenylethyl group,

$R_2$ represents a hydrogen atom, a methyl or ethyl group,

$R_3$ represents a hydrogen atom, an alkyl group with 1 to 6 carbon atoms, an allyl, propargyl, benzyl, chlorobenzyl, phenylethyl, cyclopentyl or cyclohexyl group,

$R_4$ represents a hydrogen atom, an alkyl group having 1 to 3 carbon atoms or an allyl group or

$R_3$ and $R_4$ together with the nitrogen atom between them represent a pyrrolidino, piperidino, hexamethyleneimino or morpholino group)

the enantiomers thereof and the acid addition salts thereof.

4. Tetrahydro-benzthiazoles of general formula Ia as claimed in claim 3, wherein the

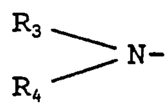

group is in the 6-position, the enantiomers thereof, and the acid addition salts thereof.

5. Tetrahydro-benzthiazoles of general formula Ia as claimed in claim 3, wherein

$R_1$ and $R_2$ together with the nitrogen atom between them represent an amino or allylamino group and

$R_3$ and $R_4$ together with the nitrogen atom between them represent a diethylamino, diethylamino, N-allyl-N-(4-chloro-benzyl)-amino, n-propylamino or pyrrolidino group, the enantiomers thereof and the acid addition salts thereof.

6. 2-Amino-6-n-propylamino-4,5,6,7-tetrahydrobenzthiazole, the enantiomers thereof, and the acid addition salts thereof.

7. (-)-2-Amino-6-n-propylamino-4,5,6,7-tetrahydro-benzthiazole, the enantiomers thereof, and the acid addition salts thereof.

8. Physiologically acceptable acid addition salts with inorganic or organic acids of the compounds as claimed in claims 1 to 7.

9. Pharmaceutical compositions containing as active substance a compound as claimed in claims 3 to 7 or a physiologically acceptable acid addition salt as claimed in claim 8 optionally together with one or more inert carriers and/or diluents.

10. Use of a compound as claimed in claims 3 to 7 or a physiologically acceptable acid addition salt as claimed in claim 8 for preparing a drug suitable for treating central nervous neuro-psychiatric diseases and/or for treating circulatory disorders.

11. Use of a compound as claimed in claims 3 to 7 or a physiologically acceptable acid addition salt as claimed in claim 8 for preparing a drug suitable for the treatment of Parkinson's disease or Parkinsonism.

12. Process for preparing a pharmaceutical composition, characterised in that a compound as claimed in claims 3 to 7 or a physiologically acceptable acid addition salt as claimed in claim 8 is incorporated in one or more inert carriers and/or diluents by a non-chemical method.

13. Process for preparing the tetrahydro-benzthiazoles as claimed in claims 1 to 8, characterised in that

a) a cyclohexanone of general formula

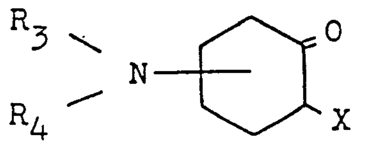

(II)

(wherein
$R_3$ and $R_4$ are defined as in claims 1 to 7 and
X represents a nucleophilically exchangeable group such as a halogen atom, e.g. a chlorine or bromine atom)
is reacted with a thiourea of general formula

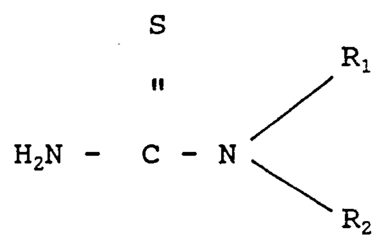

(III)

(wherein
$R_1$ and $R_2$ are defined as in claims 1 to 7),
or
b) a compound of general formula

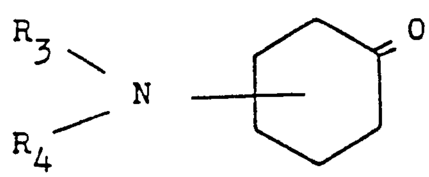

(IV)

(wherein
$R_3$ and $R_4$ are defined as in claims 1 to 7)
is reacted with a formamidine disulphide of general formula

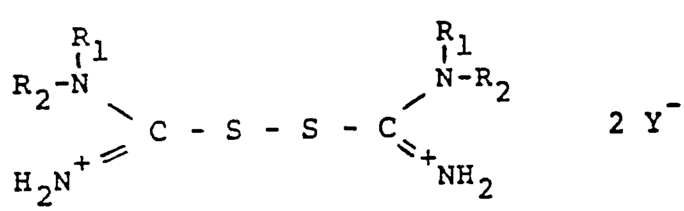

(V)

(wherein
$R_1$ and $R_2$ are defined as in claims 1 to 7 and
$Y^-$ represents an anion of an inorganic or organic acid), or
c) in order to prepare compounds of general formula I wherein at least one of the groups $R_1$, $R_2$, $R_3$ or $R_4$ represents a hydrogen atom, a protecting group is split off from a compound of general formula

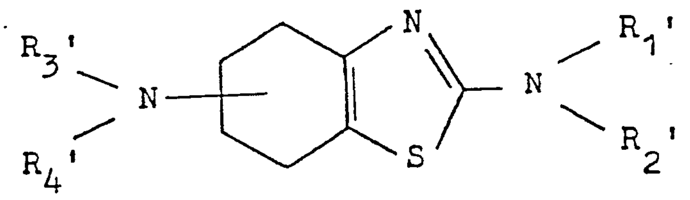

(VI)

(wherein

at least one of the groups $R_1'$, $R_2'$, $R_3'$ or $R_4'$ represents a protecting group for an amino group such as an acyl or alkoxycarbonyl group, e.g. an acetyl, propionyl, methoxycarbonyl or ethoxycarbonyl group, or $R_1'$ and $R_2'$ or $R_3'$ and $R_4'$ together with the nitrogen atom between them represent an imido group, e.g. the phthalimido group, and

the remaining groups $R_1'$, $R_2'$, $R_3'$ or $R_4'$ are as defined for $R_1$ to $R_4$ in claims 1 to 7, with the exception of the acyl groups mentioned in claims 1 and 2), or

d) in order to prepare compounds of general formula I wherein at least one of the groups $R_1$, $R_2$, $R_3$ or $R_4$ represents one of the alkyl or phenylalkyl groups mentioned in claims 1 to 7, a compound of general formula

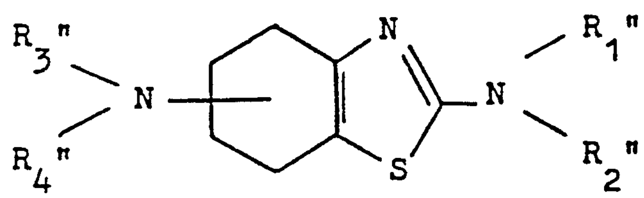

(VII)

(wherein

at least one of the groups $R_1''$, $R_2''$, $R_3''$ or $R_4''$ represents one of the acyl or phenylacyl groups mentioned in claims 1 and 2 and

the other groups have the meanings given for $R_1$, $R_2$, $R_3$ and $R_4$ in claims 1 to 7) is reduced with a metal hydride in a solvent or

e) in order to prepare compounds of general formula I wherein at least one of the groups $R_1$, $R_2$, $R_3$ or $R_4$ represents one of the alkyl, cycloalkyl, alkenyl, alkynyl or phenylalkyl groups mentioned in claims 1 to 7,

a compound of general formula

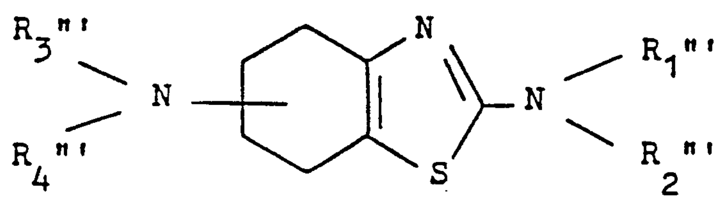

(VIII)

(wherein

at least one of the groups $R_1'''$, $R_2'''$, $R_3'''$ or $R_4'''$ represents a hydrogen atom and

the other groups $R_1'''$, $R_2'''$, $R_3'''$ or $R_4'''$ have the meanings given for $R_1$ to $R_4$ in claims 1 to 7)

is reacted with a compound of general formula

$$R_5 - Z \qquad (IX)$$

(wherein

$R_5$ represents one of the alkyl, cycloalkyl, alkenyl, alkynyl or phenylalkyl groups mentioned for $R_1$ to $R_4$ in claims 1 to 7 and

Z represents a nucleophilically exchangeable group such as a halogen atom or a sulphonic acid group, e.g. a chlorine, bromine or iodine atom, a methoxysulphonyloxy or a p-toluenesulphonyloxy group,

or Z together with an adjacent hydrogen of the group $R_5$ represents an oxygen atom),

and subsequently, if desired, a compound of general formula I thus obtained wherein at least one of the groups $R_1$ or $R_3$ represents a hydrogen atom, is converted by acylation into a corresponding compound of general formula I wherein at least one of the groups $R_1$ or $R_3$ represents one of the acyl groups mentioned in claims 1 and 2, or

a compound of general formula I thus obtained which contains at least one chiral centre is resolved into its enantiomers or

a compound of general formula I thus obtained is converted into the acid addition salts thereof, particularly the physiologically acceptable acid addition salts with organic or inorganic acids.

**Revendications**

1. Tétrahydro-benzothiazoles de formule générale

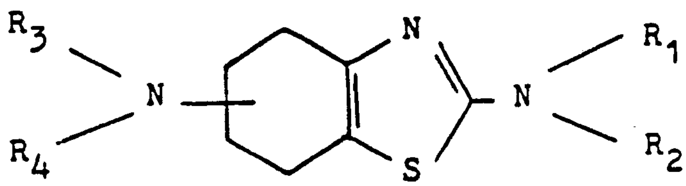

,(I)

dans laquelle

$R_1$ représente un atome d'hydrogène, un groupe alcoyle avec 1 à 6 atomes de carbone, un groupe alcényle ou alcynyle avec chacun 3 à 6 atomes de carbone, un groupe alcanoyle avec 1 à 6 atomes de carbone, un groupe phénylalcoyle ou phénylalcanoyle avec chacun 1 à 3 atomes de carbone dans la partie alcoyle, les noyaux phényle mentionnés ci-dessus pouvant chacun être substitués par 1 ou 2 atomes d'halogène,

$R_2$ représente un atome d'hydrogène ou un groupe alcoyle avec 1 à 4 atomes de carbone,

$R_3$ représente un atome d'hydrogène, un groupe alcoyle avec 1 à 7 atomes de carbone, un groupe cycloalcoyle avec 3 à 7 atomes de carbone, un groupe alcényle ou alcynyle avec chacun 3 à 6 atomes de carbone, un groupe alcanoyle avec 1 à 7 atomes de carbone, un groupe phénylalcoyle ou phénylalcanoyle avec chacun 1 à 3 atomes de carbone dans la partie alcoyle, le noyau phényle pouvant être substitué par des atomes de fluor, de chlore ou de brome,

$R_4$ représente un atome d'hydrogène, un groupe alcoyle avec 1 à 4 atomes de carbone, un groupe alcényle ou alcynyle avec chacun 3 à 6 atomes de carbone, ou

$R_3$ et $R_4$, ensemble avec l'atome d'azote intermédiaire, représentent un groupe pyrrolidino, pipéridino, hexaméthylènimino ou morpholino, leurs énantiomères et leurs sels d'addition d'acides.

2. Tétrahydro-benzothioazoles de formule générale I selon la revendication 1, dans laquelle le groupe

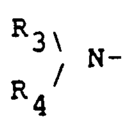

est en position 5 ou 6, leurs énantiomères et leurs sels d'addition d'acides.

3. Tétrahydro-benzothiazoles de formule générale

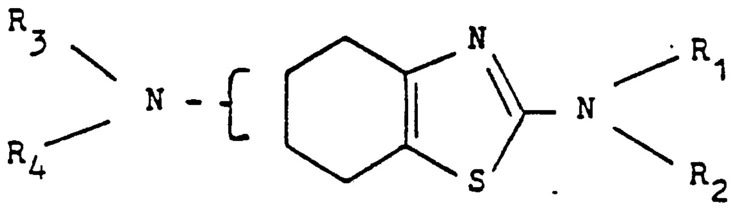 ,(Ia)

dans laquelle

$R_1$ représente un atome d'hydrogène, un groupe alcoyle avec 1 à 3 atomes de carbone, un groupe allyle, benzyle, 2-chloro-benzyle, 4-chloro-benzyle, 3,4-dichloro-benzyle ou phényléthyle,

$R_2$ représente un atome d'hydrogène, un groupe méthyle ou éthyle,

$R_3$ représente un atome d'hydrogène, un groupe alcoyle avec 1 à 6 atomes de carbone, un groupe allyle, propargyle, benzyle, chlorobenzyle, phényléthyle, cyclopentyle ou cyclohexyle,

$R_4$ représente un atome d'hydrogène, un groupe alcoyle avec 1 à 3 atomes de carbone ou un groupe allyle ou

$R_3$ et $R_4$, ensemble avec l'atome d'azote intermédiaire, représentent un groupe pyrrolidino, pipéridino, hexaméthylènimino ou morpholino, leurs énantiomères et leurs sels d'addition d'acides.

4. Tétrahydro-benzothiazoles de formule générale Ia selon la revendication 3, dans laquelle le groupe

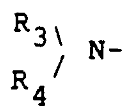

est en position 6, leurs énantiomères et leurs sels d'addition d'acides.

5. Tétrahydro-benzothiazoles de formule générale Ia selon la revendication 3, dans laquelle

$R_1$ et $R_2$, ensemble avec l'atome d'azote intermédiaire, représentent un groupe amino ou allylamino, et

$R_3$ et $R_4$, ensemble avec l'atome d'azote intermédiaire, représentent un groupe diméthylamino, diéthylamino, N-allyl-N-(4-chloro-benzyl)-amino, n-propylamino ou pyrrolidino, leurs énantiomères et leurs sels d'addition d'acides.

6. Le 2-amino-6-n-propylamino-4,5,6,7-tétrahydro-benzothiazole, ses énantiomères et ses sels d'addition d'acides.

7. Le (-)-2-amino-6-n-propylamino-4,5,6,7-tétrahydro-benzothiazole et ses sels d'addition d'acides.

8. Sels d'addition d'acides physiologiquement supportables avec des acides minéraux ou organiques des composés selon les revendications 1 à 7.

9. Médicament contenant, en tant que substance active, un composé selon les revendications 3 à 7 ou un sel d'addition d'acide physiologiquement supportable selon la revendication 8, éventuellement conjointement à un ou plusieurs excipients et/ou diluants inertes.

10. Utilisation d'un composé selon les revendications 3 à 7 ou d'un sel d'addition d'acide physiologiquement supportable selon la revendication 8 pour la fabrication d'un médicament qui convient au traitement de maladies neuropsychiatriques du système nerveux central et/ou au traitement des maladies circulatoires.

11. Utilisation d'un composé selon les revendications 3 à 7 ou d'un sel d'addition d'acide physiologiquement supportable selon la revendication 8 pour la fabrication d'un médicament qui convient au traitement de la maladie de Parkinson ou du Parkinsonisme.

12. Procédé pour la fabrication d'un médicament, caractérisé en ce qu'on introduit, par voie non chimique, un composé selon les revendications 3 à 7 ou un sel d'addition d'acide physiologiquement supportable selon la revendication 8, dans un ou plusieurs excipients et/ou diluants inertes.

13. Procédé pour la préparation des tétrahydrobenzothiazoles selon les revendications 1 à 8, caractérisé en ce que

a) on fait réagir une cyclohexanone de formule générale

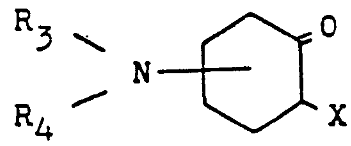

, (II)

dans laquelle
$R_3$ et $R_4$ sont définis comme dans les revendications 1 à 7, et
X représente un groupe partant nucléophile tel qu'un atome d'halogène, par exemple un atome de chlore ou de brome,
avec une thio-urée de formule générale

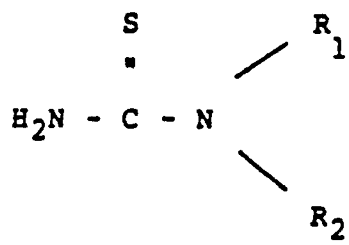

, (III)

dans laquelle
$R_1$ et $R_2$ sont définis comme dans les revendications 1 à 7, ou
b) on fait réagir un composé de formule générale

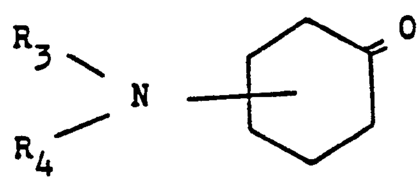

, (IV)

dans laquelle
$R_3$ et $R_4$ sont définis comme dans les revendications 1 à 7,
avec un bisulfure de formamidine de formule générale

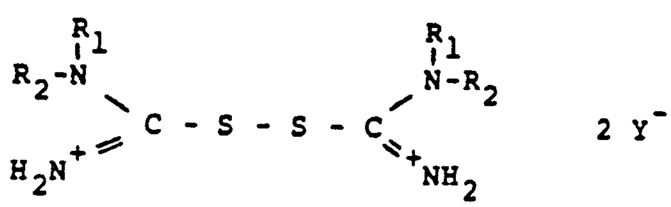

, (V)

dans laquelle
$R_1$ et $R_2$ sont définis comme dans les revendications 1 à 7, et
$Y^-$ représente un anion d'un acide minéral ou organique,
ou
c) pour la préparation de composés de formule générale I dans laquelle au moins l'un des radicaux $R_1$, $R_2$, $R_3$ ou $R_4$ représente un atome d'hydrogène, on clive un radical protecteur à partir d'un composé de formule générale

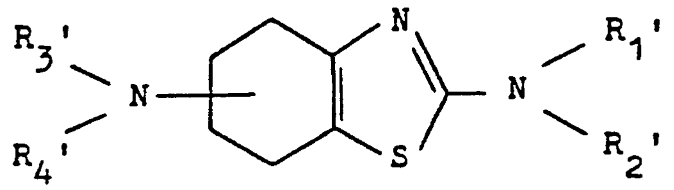

,(VI)

dans laquelle

au moins l'un des radicaux $R'_1$, $R'_2$, $R'_3$ ou $R'_4$ représente un groupe protecteur pour un groupe amino, tel qu'un groupe acyle ou alcoxycarbonyle, par exemple un groupe acétyle, propionyle, méthoxycarbonyle ou éthoxycarbonyle, ou également soit $R'_1$ et $R'_2$ soit $R'_3$ et $R'_4$, ensemble avec l'atome d'azote intermédiaire, représentent un radical imido, par exemple le radical phtalimido, et

les autres radicaux $R_1'$, $R_2'$, $R_3'$ ou $R_4'$ représentent les significations mentionnées pour $R_1$ à $R_4$ dans les revendications 1 à 7, à l'exception de la signification des radicaux acyle mentionnés dans les revendications 1 et 2, ou

d) pour la préparation de composés de formule générale I dans laquelle au moins l'un des radicaux $R_1$, $R_2$, $R_3$ ou $R_4$ représente l'un des groupes alcoyle ou phénylalcoyle mentionnés dans les revendications 1 à 7, on réduit un composé de formule générale

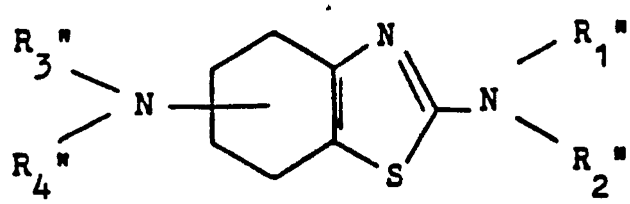

,(VII)

dans laquelle

au moins l'un des radicaux $R''_1$, $R''_2$, $R''_3$ ou $R''_4$ représente l'un des radicaux acyle ou phénylacyle mentionnés dans les revendications 1 et 2, et

les autres radicaux possèdent les significations mentionnées dans les revendications 1 à 7 pour $R_1$, $R_2$, $R_3$ et $R_4$, au moyen d'un hydrure métallique dans un solvant, ou

e) pour la préparation de composés de formule générale I dans laquelle au moins l'un des radicaux $R_1$, $R_2$, $R_3$ ou $R_4$ représente l'un des groupes alcoyle, cycloalcoyle, alcényle, alcynyle ou phénylalcoyle mentionnés dans les revendications 1 à 7, on fait réagir un composé de formule générale

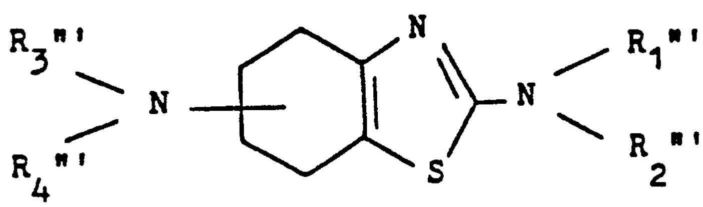

,(VIII)

dans laquelle

au moins l'un des radicaux $R'''_1$, $R'''_2$, $R'''_3$ ou $R'''_4$ représente un atome d'hydrogène et les autres radicaux $R'''_1$, $R'''_2$, $R'''_3$ et $R'''_4$ possèdent les significations mentionnées pour $R_1$ à $R_4$ dans les revendications 1 à 7, avec un composé de formule générale

$R_5$ - Z (IX)

dans laquelle

$R_5$ représente l'un des groupes alcoyle, cycloalcoyle, alcényle, alcynyle ou phénylalcoyle mentionnés dans les revendications 1 à 7 pour $R_1$ à $R_4$, et

Z représente un groupe partant nucléophile tel qu'un atome d'halogène ou un reste d'acide sulfonique, par exemple un atome de chlore, de brome ou d'iode, un groupe méthoxysulfonyloxy ou p-toluène-sulfonyloxy,

ou Z ensemble avec un atome d'hydrogène voisin du radical $R_5$ représente un atome d'oxygène,

et si on le désire ensuite, on transforme un composé ainsi obtenu de formule générale I dans laquelle au moins l'un des radicaux $R_1$ ou $R_3$ représente un atome d'hydrogène, au moyen d'une acylation correspondante, en un composé correspondant de formule générale I dans laquelle au moins l'un des radicaux $R_1$ ou $R_3$ représente l'un des radicaux acyle mentionnés dans les revendications 1 et 2, ou

on sépare un composé ainsi obtenu de formule générale I, qui contient au moins un centre chiral, en ses énantiomères ou

on transforme un composé ainsi obtenu de formule générale I en ses sels d'addition d'acides, en particulier en ses sels d'addition d'acides physiologiquement supportables avec des acides organiques ou minéraux.